(19) Europäisches Patentamt / European Patent Office / Office européen des brevets

(11) **EP 3 588 083 B1**

(12) **FASCICULE DE BREVET EUROPEEN**

(45) Date de publication et mention de la délivrance du brevet:
**02.11.2022 Bulletin 2022/44**

(51) Classification Internationale des Brevets (IPC):
**G01N 33/24** *(2006.01)*    **G01N 25/00** *(2006.01)*
**G01N 31/12** *(2006.01)*

(21) Numéro de dépôt: **19179110.2**

(22) Date de dépôt: **07.06.2019**

(52) Classification Coopérative des Brevets (CPC):
**G01N 33/24; G01N 25/00; G01N 31/12**

(54) **PROCÉDÉ POUR LA QUANTIFICATION DU SOUFRE PYRITIQUE D'UN ÉCHANTILLON DE ROCHE**

VERFAHREN FÜR DIE QUANTIFIZIERUNG VON PYRITSCHWEFEL EINER GESTEINSPROBE

METHOD FOR QUANTIFYING THE PYRITIC SULPHUR OF A ROCK SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2018 FR 1856042**

(43) Date de publication de la demande:
**01.01.2020 Bulletin 2020/01**

(73) Titulaire: **IFP Energies nouvelles**
**92852 Rueil-Malmaison Cedex (FR)**

(72) Inventeurs:
- **ABOUSSOU, Anabel**
  **92852 Rueil-Malmaison Cedex (FR)**
- **LAMOUREUX-VAR, Violaine**
  **92852 Rueil-Malmaison Cedex (FR)**
- **PILLOT, Daniel**
  **92852 Rueil-Malmaison Cedex (FR)**
- **KOWALEWSKI, Isabelle**
  **92852 Rueil-Malmaison Cedex (FR)**
- **GARCIA, Bruno**
  **92852 Rueil-Malmaison Cedex (FR)**
- **WAGNER, Thomas**
  **Edinburgh, Scotland, EH14 4AS (GB)**
- **MÄRZ, Christian**
  **Leeds, LS 12 2JB (GB)**

(56) Documents cités:
**FR-A1- 2 937 737**

- **LORANT F ET AL: "Characterization of sulfur in reservoir rocks by rock-eval analysis", 13TH EUROPEAN SYMPOSIUM ON IMPROVED OIL RECOVERY 2005 - 2005EUROPEAN ASSOCIATION OF GEOSCIENTISTS AND ENGIN,, 2005, pages 1-8, XP009178226, ISBN: 978-1-62276-890-5**

## Description

La présente invention concerne le domaine technique de l'industrie pétrolière, et plus particulièrement le domaine de l'exploration et de l'exploitation d'une formation géologique dans laquelle sont piégés des hydrocarbures.

Plus précisément, la présente invention concerne la caractérisation et la quantification du soufre présent au sein d'une roche sédimentaire, telle qu'une argile marine riche en matière organique.

Afin de répondre à la demande énergétique croissante, l'industrie pétrolière se tourne de plus en plus vers la production de pétroles bruts non conventionnels, qui sont plus riches en soufre que les pétroles conventionnels. Or la teneur en soufre d'un pétrole brut non conventionnel, ainsi que le type de composés organiques soufrés qu'il contient, sont des paramètres clés de la qualité de ce pétrole et des produits de raffinage qui en sont issus. De plus, les règlementations imposent des teneurs en soufre de plus en plus faibles pour les produits issus du raffinage. Pour ces raisons, il est important de savoir caractériser et quantifier précisément le soufre présent dans les roches qui sont à l'origine de ces pétroles bruts soufrés.

Dans le cas de roches mères pétrolières, les deux principaux composés soufrés sont le soufre organique et le soufre pyritique. La quantification du soufre organique, indépendamment du soufre pyritique, est d'une grande importance en exploration pétrolière, car elle permet de connaître exactement la quantité de soufre associée à la matière organique des roches mères, qui est à l'origine du soufre présent dans le pétrole généré par ces roches mères. En particulier, la quantification distincte du soufre pyritique et du soufre organique permet de :

- caractériser le type de matière organique de la roche mère et prévoir la qualité du pétrole généré par la roche mère en ce qui concerne sa teneur en soufre : en effet, la caractérisation du type de matière organique des roches mères se fait classiquement en fonction du contenu élémentaire de cette matière organique en Carbone (C), en Hydrogène (H) et en Oxygène (O). Cette caractérisation classique du type de matière organique se fait à l'aide du diagramme classique de Van Krevelen, qui représente le rapport atomique Hydrogène/Carbone (H/C), en fonction du rapport atomique Oxygène/Carbone (O/C). Le potentiel d'une matière organique à générer du pétrole dépendant de sa composition en H, C et O, ce diagramme permet de distinguer trois types de matière organique selon leur potentiel pétrolier. En effet, ce diagramme peut être corrélé à l'origine et à l'environnement de dépôt de la matière organique. Classiquement, on distingue la matière organique de type lacustre (type I), marine (type II) et terrestre (type III). La quantification du soufre de la matière organique indépendamment du soufre de la pyrite (ou soufre pyritique) fournit un paramètre supplémentaire qui permet une plus fine caractérisation du type de matière organique et donc une plus fine caractérisation de son environnement de dépôt et du type de pétrole qu'elle peut générer. Cette caractérisation plus fine se fait avec le diagramme de Van Krevelen étendu à trois dimensions : H/C en fonction de O/C et de $S^{org}/C$ où $S^{org}$ est la teneur en soufre organique. Ce diagramme étendu permet de distinguer avec plus de finesse les différents types de matière organique, en particulier d'identifier les matières organiques de type IS et IIS qui ont les mêmes origines que les types I et II, mais contenant du soufre, et qui se sont probablement déposées en environnement anoxique ou euxinique. Cette présence de soufre indique aussi que le pétrole résultant du craquage de cette matière organique sera plus soufré. De manière générale, les informations sur le type de matière organique de la roche mère renseignent sur le potentiel de la roche mère à générer du pétrole et sur la qualité attendue de ce pétrole notamment en ce qui concerne sa teneur en soufre ;
- apporter un paramètre supplémentaire à la corrélation huile-roche mère : en effet, la corrélation huile-roche mère est une étude très importante que doit réaliser l'homme du métier pour évaluer le système pétrolier. Elle consiste à faire le lien entre les huiles contenues dans un réservoir et la ou les roches mères qui ont généré ces huiles. Sachant que le craquage des roches mères contenant de la matière organique riche en soufre aboutit à la formation d'huiles et de gaz aussi riches en soufre, une méthode de quantification du soufre de la matière organique, indépendamment du soufre de la pyrite, fournit donc un paramètre clé pour la corrélation huile-roche mère.

## État de la technique

Les documents suivants seront cités dans la suite de la description :

Acholla, F.V., Orr, W.L., 1993. Pyrite removal from kerogen without altering organic matter: The chromous chloride method. Energy Fuels 7, 406-410.

Behar, F., Beaumont, V., De B. Penteado, H. L., 2001. Rock-Eval 6 Technology: Performances and Developments. Oil & Gas Science and Technology - Rev. IFP 56, 111-134.

Bolin, T.B., 2010. Direct determination of pyrite content in Argonne premium coals by the use of sulfur X-ray near edge absorption spectroscopy (S-XANES). Energy and Fuels 24, 5479-5482.

Canfield, D.E., Raiswell, R., Westrich, J.T., Reaves, C.M., Berner, R.A., 1986. The use of chromium reduction in the analysis of reduced inorganic sulfur in sediments and shales. Chemical Geology 54, 149-155.

Landais, P., Michels, R., Benkhedda, Z., Kister, J., Dereppe, J.-M., 1991. Behavior of Oxidized Type II Kerogen during Artificial Maturation. Energy and Fuels 5, 860-866.

Orr W., 1986, "Kerogen/asphaltene/sulfur relationships in sulfur-rich Monterey oils", Org. Geochem. Vol. 10, pp. 499-516, 1986.

Vairavamurthy, M.A., Maletic, D., Wang, S., Manowitz, B., Eglinton, T., Lyons, T., 1997. Characterization of sulfur-containing functional groups in sedimentary humic substances by X-ray absorption near-edge structure spectroscopy. Energy and Fuels 11, 546-553.

Vandenbroucke, M., Largeau, C., 2007. Kerogen origin, evolution and structure. Organic Geochemistry 38, 719-833.

On connaît des méthodes de laboratoire permettant de quantifier de manière distincte le soufre pyritique du soufre organique, telles que les techniques suivantes :

• Analyse élémentaire des kérogènes, telle que décrite dans le document (Vandenbroucke and Largeau, 2007). Il s'agit de l'une des méthodes les plus couramment utilisées en routine dans les laboratoires. Elle se déroule en 2 étapes :

- Isolation du kérogène (ou encore isolation de la matière organique) : le kérogène est isolé de la roche brute par une série d'attaques chimiques à l'acide chlorhydrique et fluorhydrique visant à détruire la matrice minérale, les carbonates et les silicates. La pyrite ($FeS_2$), d'autres sulfures métalliques, ainsi que certains oxydes mineurs dont des oxydes de fer, étant résistants à ces différentes attaques chimiques, restent conservés dans le résidu organique obtenu. Ainsi on obtient un kérogène débarrassé de la matrice minérale mais contenant encore de la pyrite.
- Analyse élémentaire du fer (par spectrométrie d'émission atomique avec plasma couplé par induction, connue sous le terme ICP-AES en anglais) et du soufre (par analyse infrarouge) : on fait ici l'hypothèse que le fer présent dans le kérogène obtenu, ne serait que sous forme de pyrite ($FeS_2$). Par conséquent à partir de la mesure de la teneur en fer (ICP-AES) du kérogène, la teneur en pyrite peut être calculée stœchiométriquement, permettant de déterminer ainsi la teneur en soufre pyritique. Puis à partir de la mesure de la teneur en soufre (IR) du kérogène on en déduit la teneur en soufre organique par différence entre le soufre total (mesuré par infrarouge) et le soufre pyritique.

Cette première méthode de laboratoire selon l'art antérieur présente les inconvénients suivants :

- la longueur du temps d'analyse : environ une semaine ;
- elle nécessite des étapes de préparation, de séparation chimique, qui sont lourdes, dangereuses car utilisant des acides forts ;
- elle ne permet pas l'automatisation des mesures ;
- elle repose sur l'hypothèse que tout le fer contenu dans la matière organique est pyritique. Or si le fer contenu dans la matière organique se trouve aussi sous d'autres formes telles que des oxydes ou d'autres sulfures que $FeS_2$, alors on surestime la teneur en soufre pyritique et l'on sous-estime la teneur en soufre organique.

• Extraction de la pyrite par le chlorure de chrome II et par l' analyse élémentaire de la roche de départ, telle que décrit dans les documents (Canfield et al., 1986; Acholla et Orr, 1993). Selon cette approche, une attaque chimique à l'acide chlorhydrique (HCl) à chaud est réalisée dans un premier temps pour extraire tout le soufre volatile contenu dans des échantillons de roches. Une fois cette étape réalisée, les échantillons sont ensuite traités, à chaud, avec une solution constituée d'acide chlorhydrique (HCl) et de chlorure de chrome II ($CrCl_2$) permettant d'extraire la pyrite ($FeS_2$). L'effluent soufré ($H_2S$), libéré par la réduction de la pyrite par cette solution, passe dans un piège composé d'une solution de nitrate d'argent ($AgNO_3$), où il précipite sous forme de sulfure d'argent ($Ag_2S$). Le précipité d'$Ag_2S$ obtenu est pesé, ce qui permet de quantifier stœchiométriquement la teneur en soufre pyritique, en supposant que la pyrite a été entièrement transformée en sulfure d'argent. Puis la teneur en soufre organique est déduite par différence entre la teneur en soufre total, obtenue par l'analyse élémentaire de la roche de départ, et la teneur en soufre pyritique. Cette méthode repose sur l'hypothèse que toute la pyrite est réduite en $H_2S$.
Cette deuxième méthode de laboratoire selon l'art antérieur présente les inconvénients suivants :

- elle nécessite des étapes de préparation, de séparation chimique, qui sont lourdes, dangereuses car utilisant des acides forts ;
- elle ne permet pas l'automatisation des mesures ; et
- elle repose sur l'hypothèse que toute la pyrite est réduite en H$_2$S. Si une partie de la pyrite n'est pas réduite, alors on sous-estime la teneur en soufre pyritique et l'on surestime la teneur en soufre organique. En particulier, les échantillons riches en pyrite risquent d'être dans ce cas.

• Spectroscopie de structure près du front d'absorption de rayons X du soufre (S-XANES en anglais), telle que décrite dans les documents (Vairavamurthy et al., 1997; Bolin, 2010) : selon cette approche, le S-XANES renseigne sur l'état d'oxydation des composés soufrés. Dans une analyse typique, le spectre d'un échantillon est déconvolué avec diverses combinaisons linéaires des spectres de différents standards de soufre. Le meilleur ajustement est choisi pour indiquer la composition réelle des différents composés soufrés de cet échantillon. Cette technique permet donc quantitativement de déterminer le soufre pyritique, le soufre organique et les sulfates. Dans le cas d'analyse de roches, notons qu'un broyage très fin de l'échantillon est souvent nécessaire afin de mieux quantifier le soufre pyritique, dont le pic est atténué dans les échantillons non finement broyés.

Cette troisième méthode de laboratoire selon l'art antérieur présente les inconvénients suivants :

- elle nécessite un broyage très fin des échantillons ; et
- elle nécessite l'accès à un synchrotron, qui est un équipement très lourd et très coûteux.

On connaît également le brevet EP 2342557 (US 8796035) qui concerne un dispositif et une méthode pour la caractérisation et la quantification du soufre dans un échantillon de roches sédimentaires ou de produits pétroliers. Plus précisément, la méthode décrite dans ce brevet comprend les étapes suivantes :

- on chauffe l'échantillon considéré dans un four de pyrolyse en atmosphère non oxydante,
- on oxyde une partie des effluents de pyrolyse, et on mesure en continu la quantité de SO$_2$ contenue dans cette partie des effluents oxydés,
- on transfère les résidus de pyrolyse dans un four d'oxydation et on mesure en continu la quantité de SO$_2$ contenue dans les effluents résultants de la chauffe oxydante du résidu de pyrolyse,
- et on en déduit la teneur en soufre dans l'échantillon.

Toutefois, cette méthode permet de déterminer la teneur en soufre total présent dans l'échantillon étudié, mais ne permet pas de quantifier séparément le soufre pyritique du soufre organique. En effet, cette méthode permet de quantifier la teneur en soufre total d'un échantillon de roche, via la mesure des effluents soufrés libérés par cet échantillon au cours d'une pyrolyse puis d'une oxydation. Deux profils correspondant au soufre sont ainsi obtenus : le premier pendant la phase de pyrolyse, et le second pendant la phase d'oxydation. Au niveau du signal soufre de pyrolyse, il est possible de discriminer le soufre organique du soufre minéral dû à la pyrite, car ils forment systématiquement deux pics suffisamment distincts. Cependant, en oxydation, les signaux de ces deux composés soufrés sont confondus, ce qui empêche la spéciation du soufre organique et pyritique. De plus, de nombreuses réactions chimiques se produisent dans la roche au cours de l'analyse même. Si certaines impliquent le soufre organique et/ou le soufre pyritique, alors elles sont susceptibles de modifier leurs signaux, ce qui rajoute un niveau de difficulté à la quantification du soufre organique et du soufre pyritique au moyen du procédé tel que décrit dans le brevet précité.

On connait également la demande de brevet FR 17/59447 (numéro de dépôt) qui décrit un procédé pour la quantification du soufre pyritique dans un échantillon de roche sédimentaire. Plus précisément, ce procédé selon l'art antérieur comprend au moins les étapes suivantes :

A. on chauffe ledit échantillon en atmosphère inerte, entre une première température comprise entre 100°C et 320°C et une deuxième tempé rature comprise entre 320°C et 700°C, en suivant un premier gradient de température compris entre 1°C/min et 30°C/min ;

B. on oxyde en continu au moins une partie des effluents issus de ladite chauffe dudit échantillon en atmosphère inerte, on mesure en continu une première quantité de SO$_2$ libérée en fonction du temps de ladite chauffe en atmosphère inerte, et on détermine au moins une teneur en soufre de pyrolyse $S_{Pyrol}$ et une teneur en soufre pyritique de pyrolyse $S_{Pyrol}^{Pyrit}$ à partir de ladite première quantité de SO$_2$ ;

C. on chauffe en atmosphère oxydante le résidu dudit échantillon issu de ladite chauffe en atmosphère inerte entre une troisième température comprise entre 280°C et 320°C et une quatrième température supérieure ou ég ale à 800°C, en suivant un second gradient de température compris entre 1°C/min et 30°C/min ;

D. on mesure en continu une deuxième quantité de $SO_2$ libérée en fonction du temps de ladite chauffe en atmosphère oxydante, on détermine au moins une teneur en soufre d'oxydation $S_{Oxy}$ à partir de ladite deuxième quantité de $SO_2$, et on détermine au moins une teneur en soufre total $S_{Total}$ par la somme de ladite teneur en soufre de pyrolyse $S_{Pyrol}$ et de ladite teneur en soufre d'oxydation $S_{Oxy}$ ;

Puis ce procédé selon l'art antérieur prévoit la détermination d'au moins une teneur en soufre pyritique $S^{Pyrit}$ contenu dans ledit échantillon à partir d'une formule du type :

$$S^{Pyrit} = p(\alpha, \beta, \gamma).S_{Pyrol}^{Pyrit} \quad ,$$

où $p(\alpha,\beta,\gamma)$ est une fonction de pondération dépendant d'un paramètre $\alpha$ représentant une proportion dudit soufre pyritique de pyrolyse par rapport audit soufre total, d'un paramètre $\beta$ représentant un effet de la matrice minérale sur ladite proportion, d'un paramètre $\gamma$ représentant un effet de la matrice organique sur ladite proportion, les valeurs desdits paramètres étant prédéterminées.

Par ailleurs, selon une variante préférée de ce procédé selon l'art antérieur, la fonction de pondération $p(\alpha,\beta,\gamma)$ s'écrit sous la forme :

$$p(\alpha, \beta, \gamma) = \frac{(1+\beta+\gamma)}{\alpha}$$

Toutefois, les valeurs des paramètres $\alpha$, $\beta$, et $\gamma$ doivent être prédéfinies, préalablement à la mise en œuvre du procédé selon l'invention. La demande de brevet FR 17/59447 décrit des valeurs par défaut, dont notamment des valeurs du paramètre $\gamma$ en fonction du type de matière organique présumé présent dans l'échantillon de roche sédimentaire considéré. Ainsi, selon une variante du procédé selon l'art antérieur, si l'échantillon de roche contient une matière organique d'origine lacustre et/ou marine, on peut utiliser 0 comme valeur pour le paramètre $\gamma$. Selon une autre variante du procédé selon l'art antérieur, si l'échantillon de roche contient une matière organique d'origine terrestre, la valeur du paramètre $\gamma$ peut être choisie entre 0.23 et 0.29, et vaut préférentiellement 0.26.

Ainsi, le procédé selon l'art antérieur décrit une méthode pour quantifier de manière directe le paramètre $\gamma$, en tenant compte des types standards de matière organique, qui sont des pôles purs. Or il peut par exemple s'avérer que l'échantillon de roche à analyser contienne des types de matière organique plus complexes, tels qu'issus de l'altération ou du mélange de matières organiques de type standard. En effet par des processus d'altération, une matière organique de type standard marin peut présenter la signature chimique d'une matière organique de type standard terrestre. De plus, la plupart des formations sédimentaires contiennent des mélanges des différents types standards de matière organique. Dans les environnements marins proximaux par exemple, il est possible de trouver dans les sédiments un mélange de matière organique de type terrestre et de matière organique de type marin. Dans de tels cas, la valeur du paramètre $\gamma$ préconisée par la demande de brevet FR 17/59447 ne serait pas adaptée à la roche sédimentaire étudiée.

De plus, la forme préférée du procédé selon l'art antérieur pour la fonction de pondération $p(\alpha,\beta,\gamma)$ , qui s'écrit sous la forme :

$$p(\alpha, \beta, \gamma) = \frac{(1+\beta+\gamma)}{\alpha}$$

est une formule approchée. En effet, comme cela est démontré dans l'exemple d'application décrit ci-après, cette formule, si elle donne des résultats satisfaisants pour la quantification du soufre pyritique présent dans un échantillon de roche, reste toutefois imprécise.

La présente invention vise à pallier ces inconvénients. Ainsi, la présente invention concerne une méthode pour une quantification très précise du soufre pyritique contenu dans un échantillon de roche sédimentaire, notamment à partir de mesures réalisées sur l'échantillon de roche lui-même pour quantifier l'effet de la matrice organique. De plus, la mise en œuvre du procédé selon l'invention est simple et rapide. Le procédé selon l'invention permet en outre, dans une de ses variantes, de quantifier le soufre organique présent dans l'échantillon, en sus du soufre pyritique.

**Le procédé selon l'invention**

L'invention concerne un procédé pour la quantification du soufre pyritique dans un échantillon de roche sédimentaire, dans lequel on applique au moins les étapes suivantes :

A. on chauffe ledit échantillon en atmosphère inerte, entre une première température comprise entre 80°C et 320°C et une deuxième température comprise entre 320°C et 700°C, en suivant une première séquence de températures, et on mesure en continu une quantité de composés hydrocarbonés, une quantité de CO et une quantité de $CO_2$ libérées pendant ladite première séquence de températures;

B. on oxyde en continu au moins une partie des effluents issus de ladite chauffe en atmosphère inerte dudit échantillon, on mesure en continu une quantité de $SO_2$ libérée par ladite oxydation desdits effluents en fonction du temps de ladite chauffe en atmosphère inerte, et on détermine au moins une teneur en soufre pyritique de pyrolyse $S^{Pyrit}_{Pyrol}$ à partir de ladite quantité de $SO_2$ ;

C. on chauffe en atmosphère oxydante le résidu dudit échantillon issu de ladite chauffe en atmosphère inerte entre une troisième température comprise entre 280°C et 320°C et une quatrième température supérieure ou égale à 800°C, en suivant une seconde séquence de températures, et on mesure en continu une quantité de CO et une quantité de $CO_2$ libérées pendant ladite seconde séquence de températures ;

caractérisé en ce qu'on détermine au moins une teneur en soufre pyritique $S^{Pyrit}$ contenu dans ledit échantillon à partir d'une formule du type :

$$S^{Pyrit} = S^{Pyrit}_{pyrol} * \frac{(1 + \frac{\beta}{1-\beta} + \frac{\gamma}{1-\gamma})}{\alpha}$$

où $\alpha$ est un paramètre représentant une proportion dudit soufre pyritique de pyrolyse par rapport audit soufre total, $\beta$ est un paramètre représentant un effet de la matrice minérale sur ladite proportion, $\gamma$ est un paramètre représentant un effet de la matrice organique sur ladite proportion, les valeurs desdits paramètres $\alpha$ et $\beta$ étant prédéterminées, et ledit paramètre $\gamma$ étant déterminé à partir d'une formule du type :

$$\gamma = f(\boldsymbol{OI}, \boldsymbol{HI})$$

où $f$ est une fonction d'au moins un indice d'oxygène OI et d'un indice d'hydrogène HI, ledit indice d'hydrogène HI étant fonction au moins de ladite quantité de composés hydrocarbonés mesurée pendant ladite chauffe en atmosphère inerte et desdites quantités de CO et de $CO_2$ mesurées pendant lesdites première et seconde séquence de températures, et ledit indice d'oxygène OI étant fonction au moins desdites quantités de CO et de $CO_2$ mesurées pendant lesdites première et seconde séquences de températures.

Selon l'invention, ladite fonction $f$ est une combinaison linéaire dudit indice d'oxygène OI et dudit indice d'hydrogène HI s'exprimant selon une formule du type : $\gamma = a * \boldsymbol{OI} + b * \boldsymbol{HI} + c$, où a, b et c sont des constantes prédéterminées.

Avantageusement, ladite constante a peut être comprise entre 0.28 et 0.46, et peut valoir préférentiellement 0.37.

Préférentiellement, ladite constante b peut être comprise entre -0.007 et -0.005 et peut valoir préférentiellement -0.006.

De manière préférée, ladite constante c peut être comprise entre 4.99 et 6.49 et peut valoir préférentiellement 5.74.

Selon une mise en œuvre de l'invention, on peut déterminer ledit indice d'hydrogène HI selon une formule du type :

$$HI = \frac{100 * S2}{TOC},$$

où

- S2 est une quantité de composés hydrocarbonés qui sont craqués au cours de la ladite première séquence de températures, S2 étant déterminée à partir de ladite quantité de composés hydrocarbonés libérés pendant ladite chauffe en atmosphère inerte,
- TOC est une teneur en carbone organique total dudit échantillon s'écrivant sous la forme $TOC(wt\%) = PC + RC$, où PC est une teneur en carbone organique de pyrolyse dudit échantillon déterminée à partir desdites mesures de CO et $CO_2$ libérés pendant ladite première séquence de températures, et où RC est une teneur en carbone organique résiduelle dudit échantillon déterminée à partir desdites mesures de CO et de CO2 libérés pendant ladite seconde séquence de températures.

Selon une mise en œuvre de l'invention, on peut déterminer ledit indice d'oxygène OI selon une formule du type :

$$OI = \left[\frac{100*S3CO_2}{TOC}\right],$$

où :

- S3CO$_2$ est une quantité de CO$_2$ mesurée entre ladite première température de ladite première séquence de températures et une première température intermédiaire de ladite première séquence de températures comprise entre 350°C et 450°C, et valant préférentiellement 400°C ;
- TOC est une teneur en carbone organique total dudit échantillon et s'écrit *TOC(wt%) = PC + RC,* où PC est une teneur en carbone organique de pyrolyse dudit échantillon déterminée à partir desdites mesures de CO et CO2 libérés pendant ladite première séquence de températures, et où RC est une teneur en carbone organique résiduelle dudit échantillon déterminée à partir desdites mesures de CO et de CO2 libérés pendant ladite seconde séquence de températures.

Selon une mise en œuvre de l'invention, ladite teneur en carbone organique de pyrolyse PC dudit échantillon peut être déterminée selon une formule du type :

$$PC(wt\%) = [Q * 0.083] + \left[(S3CO + \tfrac{1}{2}S3'CO) * \tfrac{12}{280}\right] + \left[S3CO_2 * \tfrac{12}{440}\right],$$

avec

- *S3CO2* est une quantité de CO$_2$ mesurée entre ladite première température de ladite première séquence de températures et une première température intermédiaire de ladite première séquence de températures comprise entre 350°C et 450°C, et valant préférentiellement 400°C ;
- *S3CO* est une quantité de CO mesurée entre ladite première température de ladite première séquence de températures et une deuxième température intermédiaire de ladite première séquence de températures comprise entre 500 et 600°C, et valant préférentiellement 550°C ;
- *S3'CO* est une quantité de CO mesurée entre ladite seconde température intermédiaire de ladite première séquence de températures et ladite deuxième température de ladite première séquence de températures ;

Selon une mise en œuvre de l'invention, ladite teneur en carbone organique résiduelle RC dudit échantillon peut être déterminée selon une formule du type :

$$RC(wt\%) = \left[S4CO_2 * \frac{12}{440}\right] + \left[S4CO * \frac{12}{280}\right],$$

où S4CO et S4CO$_2$ correspondent respectivement à une quantité de CO et de CO$_2$ mesurées entre ladite troisième température de ladite seconde séquence de températures et une température intermédiaire de ladite seconde séquence de températures comprise entre 320°C et 700°C, et valant préférentiellement 650°C.

Selon une première alternative de l'invention selon laquelle ledit échantillon est de type roche réservoir, ladite première température peut être comprise entre 100°C et 200°C.

Selon une deuxième alternative de l'invention selon laquelle ledit échantillon est de type roche mère conventionnelle ou roche mère non conventionnelle immature, ladite première température peut être comprise entre 280°C et 320°C.

Selon une troisième alternative de l'invention selon laquelle ledit échantillon peut être de type roche mère non conventionnelle à huile ou à gaz, ladite première température peut être comprise entre 80°C et 120°C.

Selon une mise en œuvre de l'invention, ledit paramètre $\alpha$ peut être compris entre 0.40 et 0.46, et peut valoir préférentiellement 0.43.

Selon une mise en œuvre de l'invention selon laquelle ledit échantillon de roche est de type argile, ledit paramètre β peut être compris entre 0.04 et 0.7, et peut valoir préférentiellement 0.38.

Selon une mise en œuvre de l'invention selon laquelle ledit échantillon de roche est de type marne, ledit paramètre β peut être compris entre 0.7 et 0.9, et peut valoir préférentiellement 0.78.

Selon une mise en œuvre de l'invention selon laquelle ledit échantillon de roche est de type calcaire, ledit paramètre β peut être compris entre 0.85 et 0.97, et peut valoir préférentiellement 0.9.

Selon une variante de mise en œuvre de l'invention, on peut mesurer en outre une quantité de $SO_2$ libérée pendant ladite seconde séquence de températures, on peut déterminer au moins une teneur en soufre de pyrolyse $S_{Pyrol}$ à partir de ladite quantité de $SO_2$ mesurée pendant ladite première séquence de températures et une teneur en soufre d'oxydation $S_{Oxy}$ à partir de ladite quantité de $SO_2$ mesurée pendant ladite seconde séquence de températures, et on peut déterminer une teneur en soufre organique $S^{org}$ à partir au moins de ladite teneur en soufre pyritique $S^{Pyrit}$, de ladite teneur en soufre de pyrolyse $S_{Pyrol}$ et de ladite teneur en soufre d'oxydation $S_{Oxy}$.

Selon une mise en œuvre de l'invention selon laquelle ladite quatrième température est comprise entre 800°C et 900°C, on peut déterminer une teneur en soufre organique $S^{Org}$ selon la formule : $S^{Org} = S_{Pyrol} + S_{Oxy} - S^{Pyrit}$.

Selon une mise en œuvre alternative de l'invention selon laquelle ladite quatrième température est supérieure à 1150°C, et est préférentiellement inférieure 1250°C, on peut déterminer en outre une teneur en soufres sulfates $S_{Oxy}^{Sulfa}$ à partir de la dite quantité de $SO_2$ mesurée pendant ladite seconde séquence de températures, et on peut en déduire une teneur en soufre organique selon la formule :

$$S^{Org} = S_{Pyrol} + S_{Oxy} - S^{Pyrit} - S_{Oxy}^{Sulfa}$$
.

D'autres caractéristiques et avantages du procédé selon l'invention, apparaîtront à la lecture de la description ci-après d'exemples non limitatifs de réalisations, en se référant aux Figures annexées et décrites ci-après.

**Présentation succincte des figures**

- la Figure 1a présente un exemple de mesure réalisée par un détecteur de $SO_2$ au cours d'une séquence de chauffe sous atmosphère inerte à laquelle est soumise un échantillon de roche.

- la Figure 1b présente un exemple de mesure réalisée par un détecteur de $SO_2$ au cours d'une séquence de chauffe sous atmosphère oxydante à laquelle est soumise un échantillon de roche.

- la Figure 2 présente des courbes représentatives de la quantité de SO2 libérée par quatre échantillons de pyrite ignée pure de masses distinctes pendant une séquence de chauffe sous atmosphère inerte.

- La Figure 3a présente un histogramme représentatif de l'effet de la matrice minérale, en fonction de la classe de mélanges de minéraux considérée.

- La figure 3b présente un histogramme représentatif de l'effet moyen des argiles, des carbonates et des formations intermédiaires sur la proportion de soufre de la pyrite libérée pendant la pyrolyse en fonction de la classe de mélanges de minéraux considérée.

- La figure 3c présente l'évolution de l'effet de la matrice minérale en fonction du carbone minéral.

- La figure 4 présente une comparaison entre l'effet organique obtenu par des analyses de mélanges constitués de pyrite et de différents types de matière organique et l'effet organique déterminé par le procédé selon l'invention,

- La figure 5a présente la teneur en soufre total déterminée par le procédé selon l'invention pour des échantillons de différent type, en fonction de la teneur en soufre total réelle de ces échantillons.

- La figure 5b présente la teneur en soufre pyritique déterminée par le procédé selon l'invention pour des échantillons de différent type, en fonction de la teneur en soufre pyritique réelle de ces échantillons.

- La figure 5c présente la teneur en soufre organique déterminée par le procédé selon l'invention pour des échantillons de différent type, en fonction de la teneur en soufre organique réelle de ces échantillons.

- La figure 5d présente la teneur en soufre pyritique déterminée par un procédé selon l'art antérieur pour des échantillons de différent type, en fonction de la teneur en soufre pyritique réelle de ces échantillons.

- La figure 5e présente la teneur en soufre organique déterminée par un procédé selon l'art antérieur pour des échantillons de différent type, en fonction de la teneur en soufre organique réelle de ces échantillons.

## Description détaillée du procédé

De façon générale, l'un des objets de l'invention concerne un procédé pour quantifier de manière précise le soufre pyritique présent dans un échantillon de roche sédimentaire. En particulier, la présente invention permet de quantifier le soufre pyritique de manière distincte du soufre organique. Avantageusement, le procédé selon l'invention permet de quantifier le soufre organique présent dans un échantillon de roche sédimentaire, en sus du soufre pyritique.

La présente invention peut s'appliquer à tout type de roches sédimentaires, contenant de la pyrite et/ou de la matière organique soufrée. En particulier, la présente invention est adaptée à des échantillons de roches mères, de roches réservoir ou de roches mères non conventionnelles (appelées « Shale Play » en anglais).

De manière générale, l'échantillon de roche peut par exemple avoir été prélevé par carottage au sein d'une formation souterraine d'intérêt ou bien résulter de déblais issus d'un forage. Avantageusement, l'échantillon tel que prélevé peut être préparé (par un lavage, un tamisage, un triage, etc.) afin d'en éliminer les impuretés (boue de forage par exemple, polluants etc.), puis est broyé à la main ou avec un broyeur mécanique.

Le procédé selon l'invention peut être avantageusement mais non limitativement mis en œuvre au moyen du dispositif ROCK-EVAL® (IFP Energies nouvelles, France), tel que décrit notamment dans le brevet EP 2342557 (US 8796035).

Le procédé selon l'invention comporte au moins les étapes suivantes :

**1. Séquence de chauffe sous atmosphère inerte (pyrolyse)**
**2. Séquence de chauffe sous atmosphère oxydante (oxydation)**
**3. Quantification du soufre pyritique**

Selon une première variante, le procédé selon l'invention peut comprendre en outre, à l'issue de l'étape 3, une quatrième étape de quantification du soufre organique.

Selon une deuxième variante, le procédé peut comprendre en outre une étape de calibration d'un ou de plusieurs paramètres requis pour la mise en œuvre de l'étape 3 ci-dessous. Cette étape de calibration peut être mise en œuvre indifféremment préalablement à l'étape 1, ou préalablement à l'étape 2, ou préalablement à l'étape 3, ou encore en parallèle de l'une de ces étapes 1 ou 2.

Les étapes 1 à 3 du procédé selon l'invention sont décrites ci-après, ainsi que les première et deuxième variantes du procédé selon l'invention.

## 1. Séquence de chauffe sous atmosphère inerte (pyrolyse)

Au cours de cette étape, l'échantillon de roche sédimentaire considéré est chauffé sous atmosphère inerte (comme par exemple sous un flux d'azote, d'hélium) selon un programme de températures prédéfinies, variables dans le temps (sous-étape 1.1 ci-dessous). De manière simultanée, au moins une partie des effluents issus de cette chauffe en atmosphère inerte sont oxydés en continu (sous-étape 1.2 ci-dessous).

### 1.1. Chauffe en atmosphère inerte

Selon l'invention, l'échantillon est chauffé par pyrolyse entre une température T1 comprise entre 80°C et 320°C, et une température T2 comprise entre 320°C et 700°C, préférentiellement 650°C, selon une séquence de tem pératures prédéterminée.

Selon une mise en œuvre de l'invention, la séquence de températures pour la chauffe en atmosphère inerte peut consister en un gradient de température (ou vitesse de chauffe) compris entre 0.1°C/min et 30°C/min, préférentielle ment entre 20°C/min et 30°C/min, et valant très préférentiellement 25°C/min. Selon une autre mise en œuvre de l'invention, la séquence de température pour la chauffe en atmosphère inerte peut comprendre au moins un palier de températures (lors duquel la température est maintenue constante) et au moins un gradient de températures (ou vitesse de chauffe), ce gradient pouvant être placé avant ou après le au moins un palier.

Selon une mise en œuvre de l'invention selon laquelle l'échantillon analysé est une roche réservoir, la température T1 est comprise entre 100° et 200°C, et vaut 180°C préférentiellement. On pourra se référer au brevet EP 0691540 B1 concernant la pertinence de cette gamme de températures pour ce type d'échantillon de roche.

Selon une mise en œuvre de l'invention selon laquelle l'échantillon analysé est une roche mère conventionnelle ou une roche mère non conventionnelle immature (telle qu'un schiste bitumineux, ou « black shale » en anglais), la température T1 est comprise entre 280°et 320°C, et vaut 300°C préférentiellement. On pourra se référer au document (Behar et al., 2001) concernant la pertinence de cette gamme de températures pour ce type d'échantillon de roche.

Selon une mise en œuvre de l'invention selon laquelle l'échantillon analysé est une roche mère non conventionnelle à huile (tel qu'un schiste à huile, ou « oil shale » en anglais) ou à gaz tel qu'un schiste à gaz, ou « gas shale » en anglais), la température T1 est comprise entre 80° et 120°C, et vaut 100°C préféren tiellement. On pourra se référer au brevet FR 3021748 (demande US 2015/0346179 ) concernant la pertinence de cette gamme de températures pour ce type d'échantillon de roche.

Selon l'invention, on mesure une quantité de composés hydrocarbonés, une quantité de monoxyde de carbone CO et une quantité de dioxyde carbone $CO_2$ libérées durant la séquence de chauffe en atmosphère inerte. Les mesures de composés hydrocarbonés peuvent être réalisées au moyen d'un détecteur à ionisation de flamme ou FID (« Flame Ionisation Detector ») et les mesures de CO et de $CO_2$ peuvent être réalisées au moyen d'une un spectrophotomètre infrarouge IR (InfraRouge).

Selon une mise en œuvre préférée de l'invention, à partir des mesures de monoxyde de carbone et en dioxyde de carbone réalisées pendant la séquence de chauffe en atmosphère inerte, on peut déterminer :

- une quantité de monoxyde de carbone S3CO, d'origine organique exclusivement : cette quantité correspond à la quantité de monoxyde de carbone mesurée entre la température T1 de la séquence de chauffe en atmosphère inerte et une première température intermédiaire T1' de cette séquence de chauffe en atmosphère inerte, T1' étant comprise entre 500 et 600°C, et valant préférentiellement 55 0°C ;

- une quantité de monoxyde de carbone S3'CO, d'origine à la fois organique et minérale : cette quantité correspond à la quantité de monoxyde de carbone mesurée entre la première température intermédiaire T1' de la séquence de chauffe en atmosphère inerte, et la température T2 de la séquence de chauffe en atmosphère inerte ;

- une quantité de dioxyde de carbone $S3CO_2$ de source organique exclusivement, mesurée entre la température T1 de la séquence en atmosphère inerte et une deuxième température intermédiaire de la séquence de chauffe en atmosphère inerte, comprise entre 350°C et 450°C, et valant préférentiellement 400°C.

Selon une mise en œuvre de l'invention, on détermine une teneur en carbone organique de pyrolyse PC selon une formule du type :

$$PC(wt\%) = [Q * 0.083] + \left[(S3CO + \tfrac{1}{2}S3'CO) * \tfrac{12}{280}\right] + \left[S3CO_2 * \tfrac{12}{440}\right].$$

où Q est la quantité de composés hydrocarbonés mesurée pendant la séquence de chauffe en atmosphère inerte.

### 1.2. Oxydation des effluents de la chauffe en atmosphère inerte

Selon l'invention, au moins une partie des effluents libérés pendant la pyrolyse sont oxydés, et ce, au fur et à mesure de leur libération. Les gaz soufrés présents dans les effluents de pyrolyse sont ainsi oxydés en $SO_2$, au fur et à mesure de leur libération. Selon une mise en œuvre de l'invention, cette oxydation des effluents de pyrolyse est réalisée au moyen d'une chambre de combustion, telle qu'un four d'oxydation, en présence d'un gaz oxygéné et éventuellement d'un catalyseur.

Selon l'invention, on mesure en continu, au fur et à mesure de la pyrolyse, le $SO_2$ ainsi généré, au moyen d'un détecteur de SO2 tel qu'un spectrophotomètre à ultra-violet (UV) ou à infra-rouge (IR). On obtient ainsi une mesure du $SO_2$ libéré au cours de la pyrolyse, en fonction du temps et/ou de la température de pyrolyse.

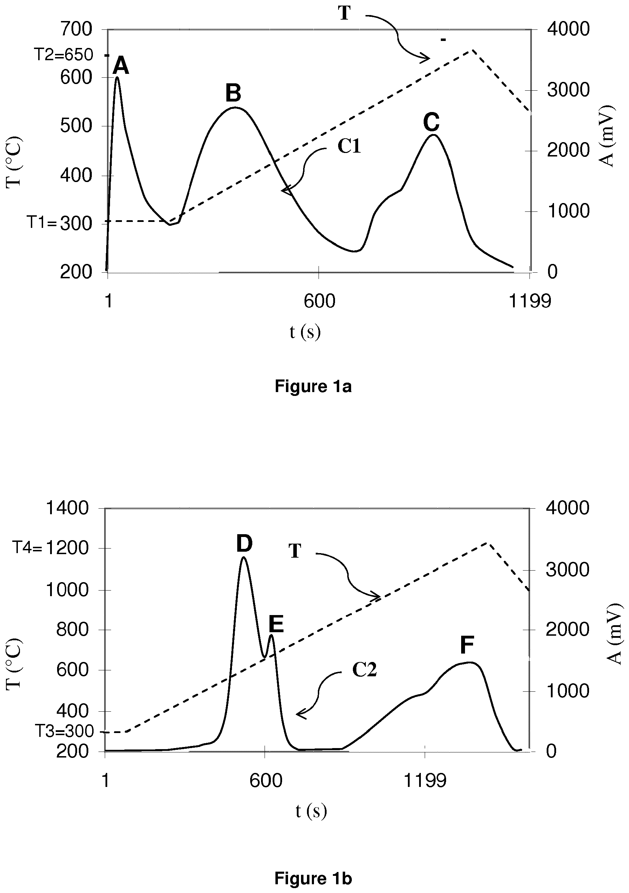

La figure 1a présente un exemple de courbe (notée C1) de mesure de la quantité de SO2 (plus précisément l'amplitude A mesurée par un détecteur de $SO_2$, tel qu'un spectrophotomètre à ultra-violet) en fonction du temps de pyrolyse (noté t), et présente également, en pointillés, l'évolution de la température de pyrolyse (notée T) en fonction du temps de pyrolyse. Pour cet exemple et à des fins illustratives, la température T1 a été choisie égale à 300°C, la température T2 a été choi sie égale à 650°C, la température T3 a été choisie égale à 300°C et la température T4 a ét é choisie égale à 1200°C. On peut observer que cette courbe C1 comporte différents pics. Notamment on peut observer sur cette courbe C1 le pic C qui correspond à la libération durant la pyrolyse d'une partie du soufre contenu dans la pyrite, dit « soufre pyritique de pyrolyse » par la suite, et noté $S_{Pyrol}^{Pyrit}$ . Par ailleurs, les deux premiers pics A et B de la courbe C1 correspondent au soufre contenu dans les composés organiques thermiquement labiles, qui sont respectivement vaporisables et thermiquement craquables.

Selon l'invention, on détermine la teneur en soufre pyritique de pyrolyse $S_{Pyrol}^{Pyrit}$ à partir de la quantité de $SO_2$ mesurée au cours de cette étape de pyrolyse. Selon une mise en œuvre de l'invention, on peut déterminer la teneur en soufre pyritique de pyrolyse $S_{Pyrol}^{Pyrit}$ à partir de l'aire sous le pic représentatif du soufre pyritique de pyrolyse sur la courbe de mesure du $SO_2$ enregistrée pendant la phase de pyrolyse (cf. pic C sur la Figure 1a), divisée par la masse de l'échantillon analysé, pondérée par un coefficient de calibration du soufre de pyrolyse. La teneur en soufre pyritique de pyrolyse est exprimée en pourcentage massique, soit en masse de soufre pyritique de pyrolyse, divisée par la masse de l'échantillon et multipliée par 100.

Selon une mise en œuvre de l'invention, on peut déterminer la teneur en soufre de pyrolyse $S_{Pyrol}$ de l'échantillon analysé à partir de l'aire sous la courbe de mesure du $SO_2$ enregistrée pendant la séquence de chauffe par pyrolyse, divisée par la masse de l'échantillon analysé, pondérée par un coefficient de calibration du soufre de pyrolyse (respectivement un coefficient de calibration du soufre d'oxydation). Ces teneurs sont exprimées en pourcentage massique, soit en masse de soufre de pyrolyse, divisée par la masse de l'échantillon et multipliée par 100.

Selon une mise en œuvre de l'invention, on peut déterminer un coefficient de calibration du soufre de pyrolyse à partir au moins d'un échantillon de référence dont on connaît la teneur en soufre, échantillon de référence que l'on soumet à une séquence de chauffe par pyrolyse. Puis on détermine le coefficient de calibration du soufre de pyrolyse à partir de l'aire sous la courbe de mesure du $SO_2$ libéré par cet échantillon de référence pendant une séquence de chauffe par pyrolyse, elle-même divisée par la masse de l'échantillon de référence. Selon une mise en œuvre de l'invention, l'échantillon de référence peut être du soufre natif pour la détermination du coefficient de calibration du soufre de pyrolyse.

**2. Séquence de chauffe en atmosphère oxydante (oxydation)**

Selon l'invention, l'échantillon est chauffé sous atmosphère oxydante entre une température T3 comprise entre 280°C et 320°C, préfé rentiellement 300°C, et une température T4 supérieure ou égale à 800°C selon un e séquence de températures prédéterminée.

Selon une mise en œuvre de l'invention, la séquence de températures pour la chauffe en atmosphère oxydante peut consister en un gradient de température (ou vitesse de chauffe) compris entre 0.1°C/min et 30°C/min, préfé rentiellement entre 20°C/min et 30°C/min, et valant très préférentiellement 25°C/mi n. Selon une autre mise en œuvre de l'invention, la séquence de température pour la chauffe en atmosphère oxydante peut comprendre au moins un palier de températures (lors duquel la température est maintenue constante) et au moins un gradient de températures (ou vitesse de chauffe), ce gradient pouvant être placé avant ou après le au moins un palier.

Selon une mise en œuvre de l'invention, cette étape peut être réalisée au moyen d'un four d'oxydation, le résidu de pyrolyse étant balayé par un flux d'air.

Selon l'invention, on mesure en continu, pendant la séquence de chauffe sous atmosphère oxydante, une quantité de monoxyde de carbone. Les mesures de CO et de $CO_2$ effectuées pendant la phase oxydante peuvent être réalisées au moyen d'une un spectrophotomètre infrarouge IR (InfraRouge).

Selon l'invention, on détermine une teneur en carbone organique résiduelle, notée RC par la suite, en fonction des mesures de CO et CO2 réalisées pendant la chauffe en atmosphère oxydante du résidu issu de la pyrolyse.

Selon une mise en œuvre de l'invention, à partir des mesures de CO et CO2 réalisées pendant la séquence de chauffe sous atmosphère oxydante, on peut déterminer au moins :

- une quantité de monoxyde de carbone, notée S4CO par la suite, qui est exclusivement de source organique : cette quantité correspond à la quantité de monoxyde de carbone libérée entre la température T3 de la séquence de chauffe en atmosphère oxydante et une température intermédiaire de la séquence de chauffe en atmosphère oxydante, comprise entre 320°C et 700°C, et valant préférenti ellement 650°C ;
- une quantité de dioxyde de carbone, notée $S4CO_2$ par la suite, qui est exclusivement de source organique : cette quantité correspond à la quantité de dioxyde de carbone mesurée entre la température T3 de la séquence de chauffe en atmosphère oxydante et une température intermédiaire de la séquence de chauffe en atmosphère oxydante, comprise entre 320°C et 700°C, et valant préférenti ellement 650°C .

Selon cette mise en œuvre de l'invention, on détermine la teneur en carbone organique résiduelle RC selon une formule du type :

$$RC(wt\%) = \left[S4CO_2 * \frac{12}{440}\right] + \left[S4CO * \frac{12}{280}\right].$$

Selon l'invention, on détermine en outre :

- un indice d'hydrogène, noté HI par la suite, qui correspond à la teneur en hydrogène de la matière organique de l'échantillon. Selon l'invention, cet indice est déterminé à partir au moins de la quantité de composés hydrocarbonés mesurées au cours de la séquence de chauffe en atmosphère inerte et quantités de CO et $CO_2$ mesurées pendant la séquence de chauffe en atmosphère inerte et pendant la séquence de chauffe en atmosphère oxydante ;
- un indice d'oxygène, noté OI par la suite, qui correspond à la teneur en oxygène de la matière organique de l'échantillon. Selon l'invention, cet indice est déterminé à partir au moins des quantités de CO et $CO_2$ mesurées pendant la séquence de chauffe en atmosphère inerte et pendant la séquence de chauffe en atmosphère oxydante.

Selon une mise en œuvre de l'invention, on peut déterminer :

- l'indice d'hydrogène HI à partir d'une formule du type :

$$HI = \frac{100*S2}{TOC},$$

où S2 correspond à une quantité de composés hydrocarbonés qui ont été craqués au cours de la chauffe de l'échantillon de roche sédimentaire en atmosphère inerte, S2 étant déterminé à partir de la quantité de composés hydrocarbonés Q libérés pendant la chauffe en atmosphère inerte, et TOC correspond à la teneur en carbone organique total, et est définie pour cette mise en œuvre de l'invention selon la formule suivante : $TOC(wt\%) = PC + RC$. Selon cette mise en œuvre de l'invention, la quantité S2 de composés hydrocarbonés qui ont été craqués au cours de la chauffe de l'échantillon de roche sédimentaire en atmosphère inerte correspond aux composés hydrocarbonés qui ne sont pas présents sous forme libre dans l'échantillon de roche considéré. Le spécialiste a parfaite connaissance de moyens pour déterminer la quantité S2 de composés hydrocarbonés qui ont été craqués au cours de la chauffe de l'échantillon de roche sédimentaire en atmosphère inerte, à partir de la quantité de composés hydrocarbonés Q libérés pendant la chauffe en atmosphère inerte, en particulier à partir d'un pyrogramme représentant l'évolution de la quantité de composés hydrocarbonés Q libérés pendant la chauffe en atmosphère inerte. En effet, un tel pyrogramme présente généralement plusieurs pics : le premier pic, souvent noté S1, correspond aux composés hydrocarbonés présents sous forme libre dans l'échantillon, le ou les autres pics suivants correspondant à la quantité de composés hydrocarbonés qui ont été craqués au cours de la chauffe de l'échantillon de roche sédimentaire en atmosphère inerte. On peut ainsi déterminer la quantité S2 à partir de la surface du ou des pics du pyrogramme différents du pic S1 ;
- l'indice d'oxygène OI à partir d'une formule du type :

$$OI = \left[\frac{100*S3CO_2}{TOC}\right].$$

où S3CO2 est la quantité de dioxyde de carbone d'origine organique mesurée pendant la séquence de chauffe en atmosphère inerte, et TOC est la teneur en carbone organique total telle que définie ci-dessus.

**3- Quantification du soufre pyritique**

Selon l'invention, au cours de cette étape, on quantifie la teneur en soufre pyritique $S^{Pyrit}$ contenu dans l'échantillon de roche sédimentaire considéré à partir du soufre pyritque de pyrolyse $S^{Pyrit}_{pyrol}$ et d'une fonction de pondération $p(\alpha,\beta,\gamma)$ selon la formule suivante (cf ci-après section « Détermination de l'expression du $S^{Pyrit}$en fonction du $S^{Pyrit}_{pyrol}$ » ci-après) :

$$S^{Pyrit} = p(\alpha, \beta, \gamma).S^{Pyrit}_{pyrol}$$

avec, selon l'invention,

$$p(\alpha, \beta, \gamma) = \frac{(1 + \frac{\beta}{1-\beta} + \frac{\gamma}{1-\gamma})}{\alpha},$$

$S^{Pyrit}$ étant exprimée en pourcentage massique, soit en masse de soufre pyritique divisée par la masse de l'échantillon et multipliée par 100, et :

- le paramètre $\alpha$, $\beta$, qui représente la proportion du soufre pyritique libérée pendant la phase de pyrolyse par rapport à son soufre total, et peut être vu comme un taux de dégradation thermique de la pyrite. Selon une mise en œuvre de l'invention, le paramètre $\alpha$ est compris entre 0.40 et 0.46, et vaut préférentiellement 0.43 ;
- le paramètre $\beta$, qui représente l'impact de la matrice minérale sur la proportion du soufre pyritique libérée pendant la phase de pyrolyse. En effet, la matrice minérale réduit la quantité de soufre de la pyrite libérée pendant la phase de pyrolyse. Selon un aspect de l'invention, le paramètre β peut être compris entre 0.04 et 0.97, en fonction du type de roche dont provient l'échantillon étudié. Selon une mise en œuvre de l'invention dans laquelle l'échantillon de roche étudié est de type argile, le paramètre $\beta$ peut être compris entre 0.04 et 0.7, et vaut préférentiellement 0.38. Selon une mise en œuvre de l'invention dans laquelle l'échantillon de roche étudié est de type marne, le paramètre $\beta$ peut être compris entre 0.7 et 0.9, et vaut préférentiellement 0.78. Selon une mise en œuvre de l'invention dans laquelle l'échantillon de roche étudié est de type calcaire, le paramètre $\beta$ peut être compris entre 0.85 et 0.97, et vaut préférentiellement 0.90.
- le paramètre γ, qui représente l'impact de la matrice organique sur la proportion du soufre pyritique libérée pendant la phase de pyrolyse, et est prédéterminé à partir d'une formule du type :

$$\gamma = f(\boldsymbol{OI}, \boldsymbol{HI})$$

où f est une fonction d'au moins l'indice d'oxygène OI et de l'indice d'hydrogène IH, ces indices ayant été déterminés au cours de l'étape 2 du procédé selon l'invention.

Selon l'invention, la fonction f est une combinaison linéaire de l'indice d'oxygène OI et de l'indice d'hydrogène HI, combinaison linéaire qui peut s'exprimer selon une formule du type : $\gamma = a * \boldsymbol{OI} + b * \boldsymbol{HI} + c$, où a, b et c sont des constantes prédéterminées. En effet, des analyses réalisées sur des échantillons variés (cf. ci-dessous, section « calibration des constantes a, b et c du paramètre $\gamma$ ») ont permis de mettre en évidence le comportement linéaire de l'effet de la matrice organique par rapport aux indices d'hydrogène et d'oxygène.

Avantageusement, la constante a est comprise entre 0.28 et 0.46, et vaut préférentiellement 0.37 et/ou la constante b est comprise entre -0.005 et -0.007 et vaut préférentiellement -0.006 et/ou la constante c est comprise entre 4.99 et 6.49 et vaut préférentiellement 5.74. En effet, des analyses réalisées sur des échantillons variés (cf. ci-dessous, section « calibration des constantes a, b et c du paramètre $\gamma$ ») ont permis de mettre en évidence le comportement linéaire de l'effet de la matrice organique par rapport aux indices d'hydrogène et d'oxygène. Avantageusement, $\gamma$ peut varier entre 0.34 (wt.%) et 74 (wt.%).

**Dispositif pour la mise en œuvre du procédé selon l'invention**

Selon une mise en œuvre de l'invention, les étapes 1 et 2 décrites ci-dessus peuvent être mises en œuvre au moyen du dispositif ROCK-EVAL® (IFP Energies nouvelles, France), développée par la demanderesse, et décrit notamment dans le brevet EP 2342557 (US 8796035). En effet, le dispositif ROCK-EVAL® comprend au moins :

- un four de pyrolyse en atmosphère non oxydante,
- des moyens d'oxydation des effluents soufrés de pyrolyse
- des moyens de mesure en continu de la quantité de $SO_2$ contenue dans lesdits effluents après oxydation, tels qu'un spectrophotomètre à ultraviolet (UV) ou à infrarouge (IR),
- des moyens de transfert des résidus de pyrolyse dans un four d'oxydation,
- un four d'oxydation en atmosphère oxydante,
- des moyens de mesure en continu de la quantité de $SO_2$ contenue dans ladite partie après oxydation, tels qu'un spectrophotomètre à ultraviolet (UV) ou à infrarouge (IR),
- des moyens de mesures des composés hydrocarbonés libérés au cours de la pyrolyse, tels qu'un détecteur à ionisation de flamme (FID),

- des moyens de mesure du monoxyde de carbone (CO) et du dioxyde de carbone ($CO_2$), tels qu'un spectrophotomètre infrarouge (IR).

Selon une alternative de mise en œuvre du procédé selon l'invention, le procédé peut également être mis en œuvre au moyen système comprenant un unique four de pyrolyse, pouvant fonctionner en atmosphère non oxydante et en atmosphère oxydante, coopérant avec des moyens de mesure de la quantité de dioxyde de soufre ($SO_2$), des moyens de mesure de la quantité de composés hydrocarbonés, ainsi que des moyens de mesures du monoxyde de carbone (CO) et du dioxyde de carbone ($CO_2$)

Variante 1 : Quantification du soufre organique

On décrit ci-après une première variante du procédé selon l'invention, visant à déterminer, en sus de la quantité de soufre pyritique présente dans l'échantillon considéré, la quantité soufre organique présente dans ce même échantillon. Pour ce faire, au cours de l'étape 2 d'oxydation du résidu de pyrolyse décrite ci-dessus, on mesure en sus le $SO_2$ généré par l'oxydation du résidu de pyrolyse et contenu dans les effluents d'oxydation. Cette mesure de $SO_2$ est par exemple réalisée au moyen d'un spectrophotomètre à UV ou IR. On obtient ainsi une mesure du $SO_2$ libéré au cours de l'oxydation, par exemple en fonction du temps et/ou de la température d'oxydation.

La figure 1b présente un exemple de courbe (notée C2) de mesure de la quantité de SO2 (plus précisément l'amplitude A mesurée par un détecteur de $SO_2$, tel qu'un spectrophotomètre à ultra-violet) en fonction du temps d'oxydation (noté t), et présente également l'évolution de la température de d'oxydation (notée T) en fonction du temps d'oxydation. Pour cet exemple et à des fins illustratives, la température T1 a été choisie égale à 300°C, la température T2 a été choisie égal e à 650°C, la température T3 a été choisie égale à 300°C et la température T4 a été c hoisie égale à 1200°C.

On peut observer que cette courbe C2 comporte différents pics. Notamment on peut observer sur la courbe C2 le pic F qui correspond à la libération du soufre contenu dans les sulfates (dit « soufre sulfates » par la suite, et noté $S_{Oxy}^{Sulfa}$) durant l'oxydation. Également, on peut observer que la courbe C2 présente deux premiers pics D et E quasi-confondus, qui correspondent respectivement à du soufre organique contenu dans des composés organiques, qui sont thermiquement réfractaires ou bien qui ont été générés pendant la phase de pyrolyse, et à du soufre pyritique. On constate ainsi que l'enregistrement du $SO_2$ libéré pendant la phase d'oxydation ne permet pas de faire la distinction entre ces deux pics et donc entre le soufre organique et le soufre pyritique.

Selon cette première variante de l'invention, on quantifie la teneur en soufre de pyrolyse $S_{Pyrol}$ libéré au cours de la pyrolyse et la teneur en soufre d'oxydation $S_{Oxy}$ libéré au cours de l'oxydation du résidu de pyrolyse à partir respectivement des mesures de SO2 réalisées pendant la séquence de chauffe en atmosphère inerte et pendant la séquence de chauffe en atmosphère oxydante. Selon cette variante de l'invention, on détermine en outre la teneur en soufre total, $S_{Total}$ comme la somme des deux teneurs $S_{Pyrol}$ et $S_{Oxy}$, soit :

$$S_{Total} = S_{Pyrol} + S_{Oxy} \,,$$

exprimée en pourcentage massique (wt.%), soit en masse de soufre total divisée par la masse de l'échantillon et multipliée par 100.

Selon une mise en œuvre de cette première variante de l'invention, on peut déterminer la teneur en soufre de pyrolyse $S_{Pyrol}$ (respectivement la teneur en soufre d'oxydation $S_{Oxy}$) de l'échantillon analysé à partir de l'aire sous la courbe de mesure du $SO_2$ enregistrée pendant la séquence de chauffe par pyrolyse (respectivement pendant la séquence de chauffe oxydante), divisée par la masse de l'échantillon analysé, pondérée par un coefficient de calibration du soufre de pyrolyse (respectivement un coefficient de calibration du soufre d'oxydation). Ces teneurs sont exprimées en pourcentage massique, soit en masse de soufre de pyrolyse (respectivement d'oxydation), divisée par la masse de l'échantillon et multipliée par 100.

Selon cette première variante de l'invention, on peut déterminer la teneur en soufre organique $S^{org}$ contenu dans l'échantillon de roche considéré à partir au moins de la différence entre la teneur en soufre total $S_{Total}$ et la teneur en soufre pyritique $S^{Pyrit}$.

Selon une première mise en œuvre de cette première variante de l'invention selon laquelle la température de fin d'oxydation T4 est comprise entre 800°C et 900°C, on peut déterminer la teneur en soufre organique $S^{org}$ contenue dans ledit échantillon selon une formule du type :

$$S^{Org} = S_{Total} - S^{Pyrit}$$

Selon une deuxième mise en œuvre de cette première variante de l'invention selon laquelle la température de fin d'oxydation T4 est comprise entre 1150°C et 1250°C, préférentiellement 1200°C , on peut déterminer la teneur en soufre organique $S^{org}$ contenu dans l'échantillon de la manière suivante :

- on quantifie une teneur en soufres sulfates $S^{Sulfa}_{Oxy}$ à partir de l'aire sous le pic représentatif du soufre sulfates de la courbe de mesure du $SO_2$ enregistrée pendant la phase d'oxydation, divisée par la masse de l'échantillon analysé, et pondérée par un coefficient de calibration du soufre d'oxydation (cf étape 3 ci-dessus pour la détermination de ce coefficient de calibration) ;
- on détermine la teneur en soufre organique $S^{org}$ selon une formule du type :

$$S^{Org} = S_{Total} - S^{Pyrit} - S^{Sulfa}_{Oxy}.$$

En effet, pour cette variante de mise en œuvre, on peut distinguer le pic $S^{Sulfa}_{Oxy}$ (cf. pic F sur la Figure 1a) qui correspond à la libération durant l'oxydation du soufre contenu dans les sulfates, survenant pour les hautes températures. La détermination de la teneur en soufre organique est plus précise selon ce deuxième mode de mise en œuvre de l'invention.

**Variante 2 : Calibration des paramètres $\alpha, \beta,$ β et $\gamma$**

Selon une mise en œuvre de l'invention, on peut calibrer les paramètres $\alpha$ et/ou $\beta$ et/ou y tels que définis ci-dessus préalablement à la mise en œuvre du procédé selon l'invention, ou bien au cours de la mise en œuvre du procédé selon l'invention, par exemple préalablement à l'étape 1, à l'étape 2 ou à l'étape 3 décrites ci-dessus, ou encore en parallèle des étapes 1 et/ou 2.

**• calibration du paramètre $\alpha$**

Selon une mise en œuvre de l'invention, on peut calibrer le paramètre $\alpha$ en estimant la proportion du soufre pyritique libéré pendant la phase de pyrolyse par rapport au soufre total à partir d'au moins un échantillon de pyrite ignée pure. Selon une mise en œuvre de l'invention, on peut obtenir une pyrite dite pure en nettoyant une pyrite naturelle de ces impuretés par des attaques chimiques.

Un exemple de calibration du paramètre $\alpha$ est décrit ci-après. Quatre échantillons issus d'un unique échantillon de pyrite ignée pure (notés respectivement E1, E2, E3, E4), de masses différentes (respectivement 2mg, 3mg, 4mg et 8mg) sont chacun soumis à une pyrolyse au moyen du dispositif ROCK-EVAL® (IFP Energies nouvelles, France). Notamment, pour cet exemple de calibration du paramètre $\alpha$, $\beta$, chaque échantillon a été placé dans le four à pyrolyse du dispositif ROCK-EVAL®, puis on a procédé à un chauffage de l'échantillon entre 300°C et 650°C, avec une rampe de température de 25°C/min et sous un flux d'azote à 150ml/min. Puis, les effluents soufrés libérés par chaque échantillon de pyrite ignée pure considéré ont été entrainés par le flux d'azote vers la chambre de combustion (four d'oxydation) du dispositif ROCK-EVAL®, où ils ont été transformés en $SO_2$ en flux continu, puis le $SO_2$ a été entraîné jusqu'à un détecteur de $SO_2$ où il a été quantifié en continu au moyen du détecteur de $SO_2$ du dispositif ROCK-EVAL®. Le résidu solide de chaque échantillon de pyrite ignée, obtenu à l'issue de la séquence de pyrolyse, a été ensuite placé dans le four à oxydation du dispositif ROCK-EVAL® puis on a procédé à un chauffage de l'échantillon entre 300°C et 850°C, av ec une rampe de température de 20°C/min et sous un flux d'air à 100ml/min. Les effluents $SO_2$ libérés ont été entraînés jusqu'à un détecteur de $SO_2$ où ils ont été quantifiés en continu au moyen du détecteur de $SO_2$ du dispositif ROCK-EVAL®.

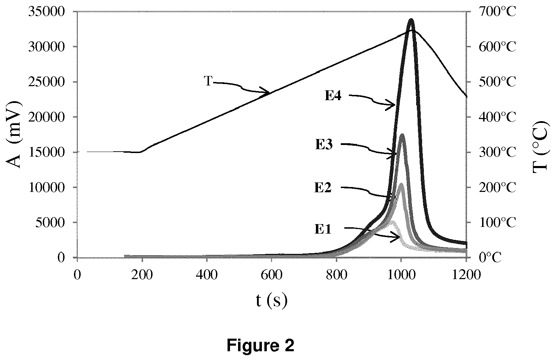

La figure 2 présente l'enregistrement dans le temps t de la quantité de $SO_2$ (plus précisément l'amplitude libéré par les échantillons E1, E2, E3, et E4 pendant la phase de pyrolyse telle que décrite ci-dessus. La courbe T présentée également sur cette figure 2 correspond à l'évolution de la température à laquelle est soumis chacun des échantillons considérés pendant cette même phase de pyrolyse. On peut notamment observer sur cette figure la présence des pics, représentatifs de la dégradation thermique de la pyrite aux différentes masses analysées pendant la phase de la pyrolyse. La teneur en soufre de pyrolyse de l'échantillon de pyrite ignée (teneur en soufre pyritique de pyrolyse) a été calculée en multipliant par la teneur en soufre de l'échantillon de référence l'aire sous chacune des courbes E1, E2, E3 et E4, divisée par la masse de l'échantillon, et ramenée à l'aire sous la courbe de mesure du $SO_2$ libéré par un échantillon de référence (tel que du soufre natif) pendant la séquence de chauffe par pyrolyse, elle-même divisée par la masse de l'échantillon de référence. Le ratio entre cette teneur en soufre pyritique de pyrolyse et la teneur en soufre total de la pyrite est calculé. Les résultats montrent que, quelle que soit la masse analysée, la proportion massique du soufre pyritique qui est libérée pendant la pyrolyse est de 0.43 ± 0.03 %wt. La proportion restante de soufre pyritique à l'issue de la pyrolyse (0.57 ± 0.03 %wt) est libérée ensuite pendant la phase d'oxydation.

Ainsi, la calibration telle que décrite ci-dessus permet de déterminer que le paramètre $\alpha$ est compris entre 0.40 et 0.46, et vaut 0.43 en moyenne.

**• calibration du paramètre** $\beta$

Selon une mise en œuvre de l'invention, on peut calibrer le paramètre $\beta$ qui représente l'impact de la matrice minérale sur la quantité du soufre de la pyrite libérée pendant la phase de pyrolyse à partir d'au moins un mélange de pyrite et d'au moins un type de minéral, ce mélange étant représentatif de l'échantillon de roche à étudier par le procédé selon l'invention.

Un exemple de calibration du paramètre $\beta$ pour différents types de minéraux est décrit ci-après. Pour cet exemple de calibration du paramètre $\beta$, on a réalisé des mélanges à partir des deux grands groupes de minéraux suivants :

- des minéraux argileux/silicatés, tels que de la

  - Silice (Sable de Fontainebleau, France) le mélange réalisé avec la silice est le mélange de référence car la silice est connu pour être non réactive ;
  - Kaolinite (Reference : CMS KGa 1b) ;
  - Smectite (Reference : Mx80) ;
  - Illite (Argile du Velay, France) : cet échantillon contenant naturellement des carbonates, il a été décarbonaté avec de l'acide chlorhydrique.

- des minéraux carbonatés, tels que de la :

  - Calcite (France) ;
  - Dolomite (Euguy, Espagne) ;
  - Sidérite (Pérou).

On réalise alors les mélanges suivants :

- 2mg de pyrite + 98mg de chaque minéral argileux/silicaté ;
- 2mg de pyrite + 58mg de chaque minéral carbonaté ;
- 2mg de pyrite + 98mg d'argiles (tous les minéraux argileux/silicatés à part égale ¼ ; ¼ ; ¼ ; ¼)) ;
- 2mg de pyrite + 58mg de carbonates (tous les minéraux carbonatés à part égale 1/3 ; 1/3 ; 1/3) ;
- 2mg de pyrite + 58mg d'argiles et de carbonates à différentes proportions, soit

  - 93% d'argiles et 7% de carbonates ;
  - 69% d'argiles et 31% de carbonates ;
  - 51% d'argiles et 49% de carbonates ;
  - 26% d'argiles et 74% de carbonates.

Ces différents échantillons sont alors soumis aux étapes 1 et 2 telles que décrites ci-dessus au moyen du dispositif ROCK-EVAL® (IFP Energies nouvelles, France). Plus précisément, chaque échantillon est placé dans le four à pyrolyse du dispositif ROCK-EVAL® puis on procède à un chauffage de l'échantillon entre 300°C et 650°C, avec une rampe de température de 25°C/min et sous un flux d'azote à 150ml/min. Selon une mise en œuvre de l'invention, les effluents soufrés libérés par chaque échantillon sont entraînés par un flux d'azote vers la chambre de combustion (four d'oxydation) du dispositif ROCK-EVAL® où ils sont transformés en $SO_2$ en flux continu, puis le $SO_2$ est entraîné jusqu'au détecteur de $SO_2$ du dispositif ROCK-EVAL® où il est quantifié en continu. Le résidu solide de chaque échantillon obtenu à l'issu de la séquence de pyrolyse est ensuite placé dans le four à oxydation du dispositif ROCK-EVAL® puis on procède à un chauffage de l'échantillon entre 300°C et 850°C, avec une rampe de température de 20 °C/min et sous un flux d'air à 100ml/min. Les effluents $SO_2$ libérés sont entraînés jusqu'à un détecteur de $SO_2$ où ils sont quantifiés en continu au moyen du détecteur de $SO_2$ du dispositif ROCK-EVAL®.

Par la suite, on appelle « effet de la matrice minérale » la grandeur s'exprimant selon une formule du type :

$$E_{Min} = \frac{S_{Pyrol}^{Pyrit,ref} - S_{pyrol}^{Pyrit,Matrix}}{S_{Pyrol}^{Pyrit,ref}} * 100,$$

où $S_{Pyrol}^{Pyrit,ref}$ est le soufre pyritique de pyrolyse libéré par un échantillon de référence (constitué de pyrite ignée pure et de silice) et $S_{pyrol}^{Pyrit,Matrix}$ est le soufre pyritique de pyrolyse libéré par un mélange considéré (pyrite ignée pure plus un minéral ou un mélange de minéraux). Pour évaluer cette grandeur, on détermine la teneur en soufre pyritique de pyrolyse, et ce, pour un échantillon de référence $S_{Pyrol}^{Pyrit,ref}$ et pour un mélange considéré $S_{Pyrol}^{Pyrit,Matrix}$.

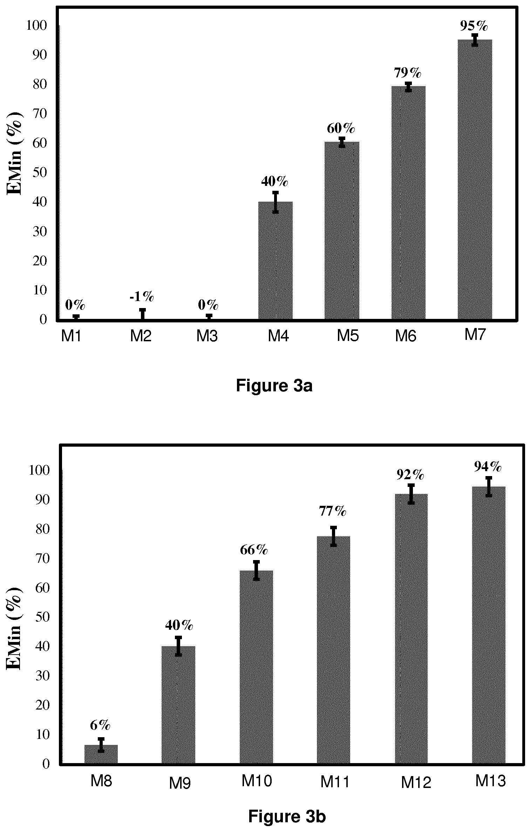

La figure 3a présente un histogramme représentatif de l'effet $E_{Min}$ de la matrice minérale en fonction de la classe de mélanges considérés dans le cas de minéraux argileux/silicatés et carbonatés, plus précisément pour les classes de mélanges suivantes :

- M1 : mélanges constitués de pyrite et de quartz (échantillon de référence) ;
- M2 : mélanges constitués de pyrite et kaolinite ;
- M3 : mélanges constitués de pyrite et d'illite ;
- M4 : mélanges constitués de pyrite et de smectite ;
- M5 : mélanges constitués de pyrite et de calcite ;
- M6 : mélanges constitués de pyrite et de dolomite ;
- M7 : mélanges constitués de pyrite et de sidérite.

La figure 3b présente un histogramme représentatif de l'effet $E_{Min}$ moyen des argiles, des carbonates et des formations intermédiaires sur la proportion de soufre de la pyrite libérée pendant la pyrolyse pour les mélanges suivants :

- M8 : mélanges constitués de 100% d'argiles ;
- M9 : mélanges constitués de 93% d'argiles et de 7% de carbonates ;
- M10 : mélanges constitués de 69% d'argiles et de 31% de carbonates ;
- M11 : mélanges constitués de 51% d'argiles et de 49% de carbonates ;
- M12 : mélanges constitués de 26% d'argiles et de 74% de carbonates ;
- M13 : mélanges constitués de 100% de carbonates.

Les figures 3a et 3b présentent aussi les barres d'erreurs pour chaque barre d'histogramme. Ces barres d'erreur ont été obtenues en estimant un écart-type établi à partir d'une répétition des analyses telles que décrites ci-dessus.

Ainsi, les résultats obtenus par la mise en œuvre de la méthode de calibration du paramètre β telle que décrite ci-dessus pour les différents mélanges décrits ci-dessus mettent en évidence le fait que la matrice minérale peut réduire la proportion de soufre de la pyrite libérée pendant la phase de pyrolyse. Toutefois, cet effet est très variable selon le type de minéral en présence. La réduction relative de la proportion de soufre libérée par la pyrite en pyrolyse varie entre 0% et 40% en présence de minéraux argileux/silicatés et entre 60% et 98% en présence de minéraux carbonatés (cf. figure 3a). L'effet moyen des argiles s'élève à 6% tandis que celui des carbonates atteint 93% (cf. figure 3b). Entre ces deux pôles on observe une évolution croissante de l'effet de la matrice minérale $E_M$ en fonction de la part d'argiles et de carbonates dans le mélange (cf. figure 3b).

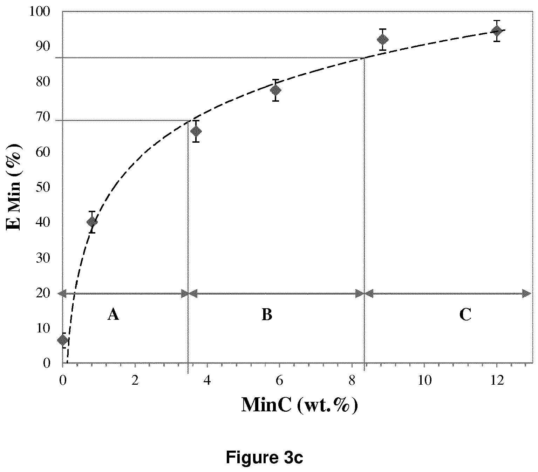

La figure 3c montre l'évolution de l'effet $E_{Min}$ de la matrice minérale en fonction du carbone minéral (noté MinC ci-après), qui est un paramètre pouvant être mesuré par exemple avec le dispositif ROCK-EVAL® (IFP Energies nouvelles, France), et qui est un indicateur de la teneur en carbonates des mélanges. On peut observer sur cette figure que le MinC varie sur une gamme comprise entre 0wt% et 12wt%, ce qui correspond à un équivalent calcite entre 0wt% et 100wt%. Grâce à ce paramètre, on peut définir trois types de lithologies : les argiles, les marnes et les calcaires. La zone (A) de la figure 3c représente la zone des argiles, qui ont des teneurs en carbonates-équivalent calcite comprises entre 0wt% et 30wt% (0≤MinC argiles<3.6wt%). Dans cette zone des formations argileuses, l'effet de la matrice sur la quantité du soufre de la pyrite libérée pendant la phase de pyrolyse varie entre 6% et 70%, avec une moyenne de 38%. La zone (B) de la figure 3c représente la zone des marnes, qui ont des teneurs en carbonates-équivalent calcite comprises entre 30% et 70% (3.6≤MinC marnes<8.4wt%). Dans cette zone des formations marneuses, la valeur moyenne d'effet de la matrice sur la quantité du soufre de la pyrite libérée pendant la phase de pyrolyse varie entre 70% et 87%, avec une moyenne de 78%. La zone (C) de la figure 3c représente la zone des calcaires qui ont des teneurs en carbonates-équivalent calcite comprises entre 70% et 100% (8.4wt%5MinC calcaires≤12wt%). Dans cette zone des formations calcaires, la valeur moyenne d'effet de la matrice sur la quantité du soufre de la pyrite libérée pendant la phase de pyrolyse varie entre 87% et 94%, avec une moyenne de 90%.

Ainsi, le paramètre β varie entre 0.06 et 0.94 selon le type de formations sédimentaires, et plus précisément, dans le cas des :

- Argiles : le paramètre β vaut en moyenne 0.38 ;
- Marnes : le paramètre β vaut en moyenne 0.78 ;
- Calcaires : le paramètre β vaut en moyenne 0.90.

**• calibration des constantes a, b, et c du paramètre $\gamma$**

Cette étape peut être mise en œuvre dans le cadre de la mise en œuvre préférée du procédé selon l'invention, selon laquelle le paramètre $\gamma$ s'écrit sous la forme :

$$\gamma = a * OI + b * HI + c \ ,$$

où a, b et c sont des constantes prédéterminées.

Selon une mise en œuvre de l'invention comprenant une étape de calibration des constantes a, b, c du paramètre $\gamma$, on peut réaliser des mélanges constitués de pyrite et de différents types de matière organique classiquement notés :

- Type I : matière organique lacustre, telle que les « green river shales » (Eocène, USA) ;
- Type II: matière organique marine, telle que les « schistes carton » du Bassin de Paris (Toarcien, France);
- Type II : matière organique marine provenant du puits ODP 959 (Coniacien-Santonien, Côte d'Ivoire-Ghana) ;
- Type II oxydé : matière organique marine, telle que les « schistes carton » du Bassin de Paris (Toarcien, France), oxydée artificiellement selon la méthode décrite dans le document (Landais *et al.,* 1991).
- Type IIS : matière organique marine riche en soufre organique, telle que le « Phosphoria Formation » (Permien, USA) ;
- Type III : matière organique terrestre, telle que le « Calvert bluff Formation » (Paléocène, USA);
- Type III oxydé : matière organique terrestre, telle que le « Calvert bluff Formation » (Paléocène, USA), oxydée artificiellement selon la méthode décrite dans le document (Landais *et al.,* 1991).
- Mélanges de types II et III : mélanges de matière organique marine, telle que les « schistes carton » du Bassin de Paris (Toarcien, France) et de matière organique terrestre, telle que le « Calvert bluff Formation » (Paléocène, USA);

Par la suite, on appelle « effet de la matrice organique » la grandeur s'exprimant selon la formule suivante :

$$E_{Org} = \frac{S_{Pyrol}^{Pyrit+MO\ obtenu} - S_{pyrol}^{Pyrit+MO\ attendu}}{S_{Pyrol}^{Pyrit+MO\ attendu}} \times 100$$ , où $S_{Pyrol}^{Pyrit+MO\ obtenu}$ est le soufre pyritique de pyrolyse obtenu après l'analyse du mélange formé de pyrite et de matière organique (telle que décrite à l'étape 1 ci-dessus) et $S_{Pyrol}^{Pyrit+MO\ attendu}$ est la valeur attendue de soufre pyritique de pyrolyse. du mélange. Cette valeur de référence théorique est calculée comme suit :

- on analyse chaque échantillon de matière organique seul, au moyen du dispositif ROCK-EVAL® (IFP Energies nouvelles, France), pour quantifier sa teneur en soufre pyritique de pyrolyse (tel que décrite à l'étape 1 ci-dessus) ;
- on analyse la pyrite seule, au moyen du dispositif ROCK-EVAL® (IFP Energies nouvelles, France), pour quantifier sa teneur en soufre pyritique de pyrolyse (tel que décrite à l'étape 1 ci-dessus) ;
- on additionne proportionnellement, en fonction du ratio pyrite/matière organique, le soufre pyritique de pyrolyse de la pyrite et le soufre pyritique de pyrolyse de la matière organique.

Par ailleurs, on détermine l'indice d'hydrogène HI et l'indice d'oxygène OI pour chacun des échantillons décrits ci-dessus, tel que décrit à l'étape 2 ci-dessus, au moyen du dispositif ROCK-EVAL® (IFP Energies nouvelles, France). En particulier, on détermine pour ce faire

- la teneur en carbone organique de pyrolyse PC selon la formule :

$$PC(wt\%) = [Q * 0.083] + \left[S3CO * \frac{12}{280}\right] + \left[S3CO_2 * \frac{12}{440}\right]$$

- la teneur en carbone organique résiduelle RC selon la formule :

$$RC(wt\%) = \left[S4CO_2 * \frac{12}{440}\right] + \left[S4CO * \frac{12}{280}\right].$$

- l'indice d'oxygène OI est déterminé selon la formule :

$$OI = \left[\frac{\left(S3CO*\frac{16}{28}\right)+\left(S3CO_2*\frac{32}{44}\right)}{RC+PC}\right]$$

- l'indice d'hydrogène IH est déterminé selon la formule : $HI = \frac{100*S2}{RC+PC}$

Puis, on effectue une régression multi-variables portant sur l'effet de la matrice organique $E_{Org}$ en fonction de l'indice d'oxygène OI et de l'indice d'hydrogène HI et on détermine les constantes a, b, et c du paramètre $\gamma$ tel que défini ci-dessus et s'exprimant sous la forme : $\gamma = a * OI + b * HI + c$ . La régression linéaire ainsi décrite permet d'obtenir la formule suivante pour le paramètre $\gamma$ représentant l'effet de la matrice organique :

$$\gamma = 0.37 * \boldsymbol{OI} - 0.006 * \boldsymbol{HI} + 5.74$$

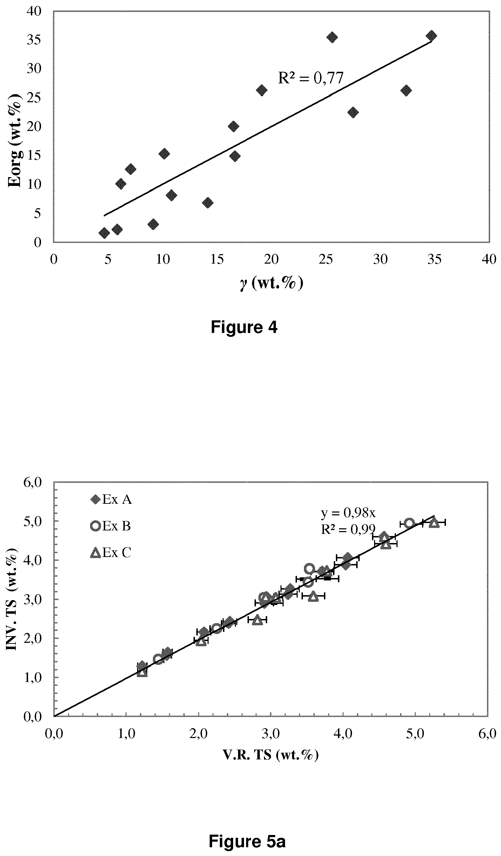

La figure 4 présente la comparaison entre la valeur de $\gamma$ ainsi déterminée par régression (droite continue) et les valeurs déterminées par mesures sur les différents échantillons (losanges) tel que décrit ci-dessus. On peut observer une bonne corrélation entre les valeurs prises par $\gamma$ et les valeurs de $E_{org}$ déterminées par mesures (le coefficient de régression linéaire R2 est de 0.77), ce qui démontre que l'on peut prédire de manière fiable l'effet de la matière organique à partir de l'indice d'oxygène OI et de l'indice d'hydrogène HI. Par ailleurs, à partir de ces expériences réalisées sur différents types d'échantillon, on définit les barres d'erreur suivantes sur chacune des constantes :

-

$$a = 0.37 \pm 0.09$$

-

$$b = -0.006 \pm 0.001$$

-

$$c = 5.74 \pm 0.75$$

Par ailleurs, sachant que généralement $0 \leq IH(mgHC/gTOC) \leq 900$ et $0 \leq IO(mgCO_2/gTOC) \leq 200$, $\gamma$ peut varier entre 0.34 wt.% (IH=900; IO=0) et 74wt.% (IH=0; IO=200).

Détermination de l'expression de la fonction de pondération

La fonction de pondération $p(\alpha, \beta, \gamma)$ du procédé selon l'invention est différente de celle de la demande de brevet FR 17/59447 (numéro de dépôt). La justification de l'expression de la fonction de pondération du procédé selon l'invention est détaillée ci-après. $S_{pyrol}^{Pyrit}$ représente une teneur en soufre pyritique de pyrolyse qui a été réduite par la présence de la matrice minérale et de la matrice organique. Il convient donc dans une première étape de corriger la teneur en soufre pyritique de pyrolyse $S_{pyrol}^{Pyrit}$ de l'effet minéral et de l'effet organique. Cela permet ensuite de quantifier le soufre pyritique de pyrolyse total $S_{pyrol\ total}^{Pyrit}$ , puis de déduire le soufre pyritique total $S^{Pyrit}$.

**Correction de l'effet minéral Corr$\beta$:**

L'effet minéral $\beta$ représente la proportion du soufre pyritique de pyrolyse qui est retenue dans la matrice minérale. Ainsi, connaissant l'effet de la matrice minérale $\beta$, on peut retrouver le soufre pyritique de pyrolyse sans cet effet de la matrice minérale $S_{Pyrol}^{Pyrit\ sans\ Emin}$. La formule de l'effet minérale peut s'écrire de la manière suivante :

$$\beta = \frac{S_{Pyrol}^{Pyrit\ sans\ Emin} - S_{pyrol}^{Pyrit}}{S_{Pyrol}^{Pyrit\ sans\ Emin}}$$

$$\beta = 1 - \frac{S_{pyrol}^{Pyrit}}{S_{Pyrol}^{Pyrit\ sans\ Emin}}$$

$$\frac{S_{pyrol}^{Pyrit}}{S_{Pyrol}^{Pyrit\ sans\ Emin}} = 1 - \beta$$

$$S_{Pyrol}^{Pyrit\ sans\ Emin} = \frac{S_{pyrol}^{Pyrit}}{1 - \beta}$$

On définit alors *Corr$\beta$,* qui représente la quantité du soufre pyritique de pyrolyse qui a été retenue dans la matrice minérale, selon la formule suivante :

$$Corr\beta = S_{Pyrol}^{Pyrit\ sans\ Emin} - S_{pyrol}^{Pyrit}$$

$$Corr\beta = \frac{S_{pyrol}^{Pyrit}}{1 - \beta} - S_{pyrol}^{Pyrit}$$

$$Corr\beta = S_{pyrol}^{Pyrit}\left(\frac{1}{1 - \beta} - 1\right)$$

$$Corr\beta = S_{pyrol}^{Pyrit}\left(\frac{\beta}{1 - \beta}\right)$$

### Correction de l'effet organique CorrE$_{org}$

L'effet organique $\gamma$ représente la proportion du soufre pyritique de pyrolyse qui est retenue dans la matrice organique. Ainsi, connaissant l'effet de la matrice organique $\gamma$, on peut retrouver le soufre pyritique de pyrolyse sans cet effet de la matrice organique $S_{Pyrol}^{Pyrit\ sans\ Eorg}$. La formule de l'effet organique peut s'écrire de la manière suivante :

$$\gamma = \frac{S_{Pyrol}^{Pyrit\ sans\ Eorg} - S_{pyrol}^{Pyrit}}{S_{Pyrol}^{Pyrit\ sans\ Eorg}}$$

$$\gamma = 1 - \frac{S_{pyrol}^{Pyrit}}{S_{Pyrol}^{Pyrit\ sans\ Eorg}}$$

$$\frac{S_{pyrol}^{Pyrit}}{S_{Pyrol}^{Pyrit\ sans\ Eorg}} = 1 - \gamma$$

$$S_{Pyrol}^{Pyrit\ sans\ Eorg} = \frac{S_{pyrol}^{Pyrit}}{1 - \gamma}$$

On définit alors $CorrE_{org}$, qui représente la quantité du soufre pyritique de pyrolyse qui a été retenue dans la matrice organique, selon la formule suivante :

$$Corr\gamma = S_{Pyrol}^{Pyrit\ sans\ Eorg} - S_{pyrol}^{Pyrit}$$

$$Corr\gamma = \frac{S_{pyrol}^{Pyrit}}{1 - \gamma} - S_{pyrol}^{Pyrit}$$

$$Corr\gamma = S_{pyrol}^{Pyrit}\left(\frac{1}{1 - \gamma} - 1\right)$$

$$Corr\gamma = S_{pyrol}^{Pyrit}\left(\frac{\gamma}{1 - \gamma}\right)$$

**Calcul du soufre pyritique de pyrolyse. total** $S_{pyrol\ total}^{Pyrit}$

Le soufre pyritique de pyrolyse total $S_{pyrol\ total}^{Pyrit}$ est alors obtenu à partir de la somme entre $S_{pyrol}^{Pyrit}$ (la teneur en soufre de pyrolyse réduite par la présence de la matrice minérale et de la matrice organique), $Corr\beta$ (la quantité du soufre pyritique de pyrolyse qui a été retenue dans la matrice minérale) et $Corr\gamma$ (la quantité du soufre pyritique de pyrolyse qui a été retenue dans la matrice organique) de la manière suivante :

$$S_{pyrol\ total}^{Pyrit} = S_{pyrol}^{Pyrit} + Corr\beta + Corr\gamma$$

$$S_{pyrol\ total}^{Pyrit} = S_{pyrol}^{Pyrit} + S_{pyrol}^{Pyrit}\left(\frac{\beta}{1 - \beta}\right) + S_{pyrol}^{Pyrit}\left(\frac{\gamma}{1 - \gamma}\right)$$

$$S_{pyrol\ total}^{Pyrit} = S_{pyrol}^{Pyrit}\left(1 + \frac{\beta}{1 - \beta} + \frac{\gamma}{1 - \gamma}\right)$$

**Calcul du soufre pyritique total** $S^{Pyrit}$

Le soufre pyritique *total* $S^{Pyrit}$ est calculé à partir soufre pyritique de pyrolyse total $S_{pyrol\ total}^{Pyrit}$ et du paramètre $\alpha$ (la proportion du soufre pyritique de pyrolyse total $S_{pyrol\ total}^{Pyrit}$ par rapport au soufre pyritique total $S^{Pyrit}$) :

$$S^{Pyrit} = \frac{S^{Pyrit}_{pyrol\ total}}{\alpha}$$

$$S^{Pyrit} = S^{Pyrit}_{pyrol} * \frac{(1 + \frac{\beta}{1 - \beta} + \frac{\gamma}{1 - \gamma})}{\alpha}$$

$$S^{Pyrit} = p(\alpha, \beta, \gamma) * S^{Pyrit}_{pyrol}$$

On en déduit donc l'expression suivante pour la fonction de pondération permettant de déterminer le soufre pyritique total $S^{Pyrit}$ à partir du soufre pyritique de pyrolyse $S^{Pyrit}_{pyrol}$ mésuré :

$$p(\alpha, \beta, \gamma) = \frac{(1 + \frac{\beta}{1 - \beta} + \frac{\gamma}{1 - \gamma})}{\alpha}$$

**Exemples d'application**

L'exemple d'application ci-après vise à évaluer la qualité des résultats obtenus par la mise en œuvre du procédé selon l'invention. Pour ce faire, on constitue différents mélanges formés à partir de neuf échantillons de roches sédimentaires ne contenant que du soufre organique et ce, en quantité connue, auxquels on ajoute des masses de pyrite connues. Les échantillons de roche proviennent de trois formations différentes (« Orbagnous », « Phosphoria » et « Limagne ») et ont été prélevés dans différents niveaux de ces formations. Les caractéristiques de ces neuf échantillons de roches sédimentaires sont résumées dans les neuf premières lignes des Tableaux 1a et 1b ci-dessous. Des masses différentes de pyrite ont été ajoutées à ces neufs échantillons, selon les caractéristiques résumées dans les lignes 10 et 11 des Tableaux 1a et 1b ci-dessous. De cette manière, on réalise 14 mélanges de type « pyrite + Orbagnoux » (type noté EXA par la suite), 6 mélanges de type « pyrite + Phosphoria » (type noté EXB par la suite), et 8 mélanges de type « pyrite + Limagne» (type noté EXC par la suite).

Puis, on détermine les teneurs en soufre pyritique et en soufre organique de chacun de ces mélanges au moyen du procédé selon l'invention d'une part, et d'autre part au moyen du procédé selon l'art antérieur décrit dans la demande de brevet FR.17/59.447.

Le procédé selon l'invention est mis en œuvre au moyen du dispositif ROCK-EVAL® (IFP Energies nouvelles, France). Plus précisément, chaque mélange est placé dans le four de pyrolyse du dispositif ROCK-EVAL® puis on procède à un chauffage du mélange entre 300°C et 650°C, avec une rampe de température de 25 °C/min et sous un flux d'azote à 150ml/min. Selon une mise en œuvre de l'invention, les effluents soufrés libérés par chaque échantillon sont entraînés par un flux d'azote vers une chambre de combustion (encore appelée four d'oxydation) du dispositif ROCK-EVAL® où ils sont transformés en $SO_2$ en flux continu, puis le $SO_2$ est entraîné jusqu'au détecteur de $SO_2$ du dispositif ROCK-EVAL® où ils sont quantifiés en continu. A l'issue de la pyrolyse, chaque résidu du mélange est transféré depuis le four de pyrolyse vers le four d'oxydation du dispositif ROCK-EVAL® puis on procède à un chauffage de l'échantillon entre 300°C et 850°C ou 1200°C selon la mise en œuvre, avec une rampe de température de 20°C/min et sous un flux d'air à 100ml/min. Les effluents de $SO_2$ libérés par cette oxydation sont entraînés jusqu'au détecteur de $SO_2$ du dispositif ROCK-EVAL® où il est quantifié en continu. Les teneurs en soufre total, en soufre pyritique et en soufre organique de chaque mélange sont déterminées par la mise en œuvre du procédé selon l'invention tel que décrit ci-dessus.

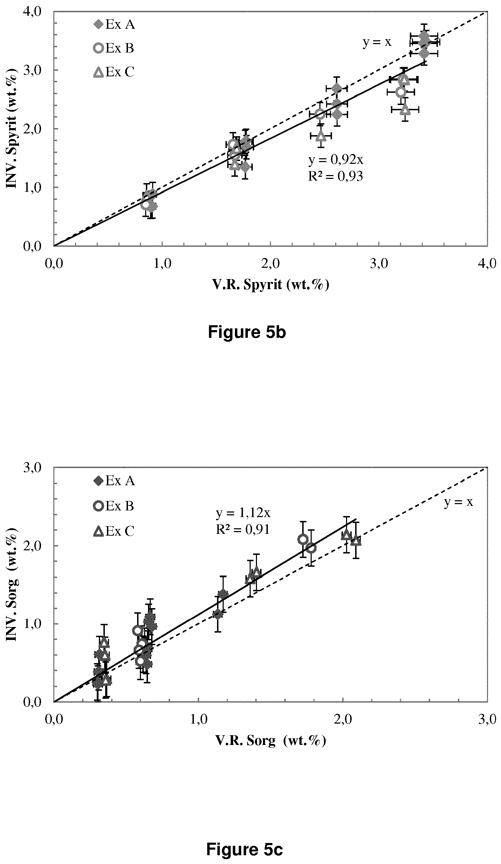

Les figures 5a, 5b, et 5c présentent respectivement l'évolution des teneurs en soufre total (INV TS), en soufre pyritique (INV $S_{pyrit}$) et en soufre organique (INV $S_{org}$) obtenues par le procédé selon l'invention, en fonction des teneurs de référence respectivement en soufre total (VR TS), en soufre pyritique (VR $S_{pyrit}$) et en soufre organique (VR $S_{org}$) pour chacun des mélanges du type EXA (soit 14 mélanges « pyrite + Orbagnoux »), pour chacun des mélanges du type EXB (soit 6 mélanges « pyrite + Phosphoria») et pour chacun des mélanges du type EXC (soit 8 mélanges « pyrite + Limagne»).

On peut observer sur les figures 5a, 5b, et 5c une très bonne corrélation entre les teneurs en soufre total, en soufre pyritique et en soufre organique déterminées à partir du procédé selon l'invention et les teneurs de référence en soufre total, en soufre pyritique et en soufre organique (corrélation avec une pente proche de 1). Cela confirme la précision de la détermination de la teneur en soufre pyritique et en soufre organique d'un échantillon par le procédé selon l'invention.

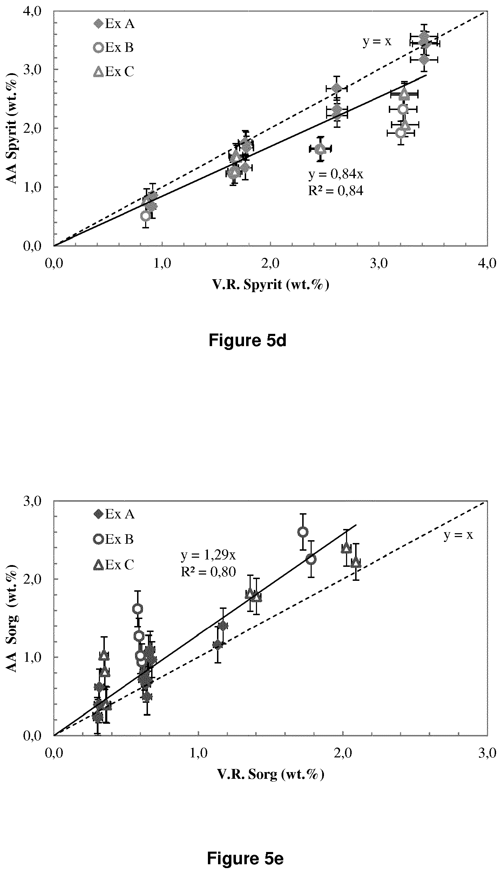

Les figures 5d, et 5e présentent respectivement l'évolution des teneurs en soufre pyritique (AA $S_{pyrit}$) et en soufre organique (AA $S_{org}$) obtenues par le procédé selon l'art antérieur en fonction des teneurs de référence respectivement en soufre pyritique (VR $S_{pyrit}$) et en soufre organique (VR $S_{org}$) pour chacun des mélanges du type EXA (soit 14 mélanges « pyrite + Orbagnoux »), pour chacun des mélanges du type EXB (soit 6 mélanges « pyrite + Phosphoria») et pour chacun des mélanges du type EXC (soit 8 mélanges « pyrite + Limagne»).

On peut observer sur les figures 5d et 5e une moins bonne corrélation entre les teneurs en soufre pyritique et en soufre organique déterminées à partir du procédé selon l'art antérieur et les teneurs de référence en soufre pyritique et en soufre organique.

Ainsi, la présente invention permet d'améliorer de manière significative la précision de la détermination de la teneur en soufre pyritique contenue dans un échantillon de roche sédimentaire, et par conséquent la précision de la détermination de la teneur en soufre organique contenue dans un échantillon de roche sédimentaire.

Tableau 1a

| Formations | Orbagnoux | | | | Phosphoria | |
|---|---|---|---|---|---|---|
| Ages | Kimmeridgien | | | | Permien | |
| Pays | France | | | | Etats-Unis | |
| Echantillons | O-9m | O-9ka | O-9cb | O-9ca | P-55 | P- 43 |
| IO (mgCO$_2$/ gCOT) | 40.0 | 25.6 | 11.6 | 24.0 | 127.5 | 61.3 |
| IH (mgHC/gCOT) | 964.6 | 969.6 | 811.8 | 887.5 | 134.5 | 372.5 |
| Teneur en carbone minéral (wt.%) | 11.2 | 10.8 | 0.8 | 10.7 | 0.3 | 0.4 |
| Masse de la roche (mg) | 60±0.02 | 60±0.02 | 60±0.02 | 30±0.02 | 60±0.02 | 30±0.02 |
| Teneur en soufre organique (wt. % ) | 0.3±0.03 | 0.7±0.03 | 0.7±0.03 | 1.21±0.03 | 0.6±0.03 | 1.8±0.03 |
| Masse de la pyrite (mg) | 1; 2; 3; 4 ±0.02 | 1; 2; 3; 4 ±0.02 | 1; 2; 3; 4 ±0.02 | 1; 2 ±0.02 | 1; 2; 3; 4 ±0.02 | 1; 2 ±0.02 |
| Teneur en soufre pyritique (wt. % ) | 53±2 | 53±2 | 53±2 | 53±2 | 53±2 | 53±2 |

Tableau 1b

| Formations | Limagne | | |
|---|---|---|---|
| Ages | Oligocène- Eocène | | |
| Pays | France | | |
| Echantillons | L-S18-2 | L-S02-5 | L-S05-2 |
| IO (mgCO$_2$/gCOT) | 39.0 | 31.5 | 30.5 |
| IH (mgHC/gCOT) | 549.0 | 660.0 | 691.5 |
| Teneur en carbone minéral (wt.%) | 0.1 | 0.2 | 0.2 |
| Masse de la roche (mg) | 60±0.02 | 30±0.02 | 30±0.02 |

# EP 3 588 083 B1

(suite)

| Formations | Limagne | | |
|---|---|---|---|
| **Teneur en soufre organique (wt. % )** | 0.4±0.03 | 2.2±0.03 | 1.5±0.03 |
| **Masse de la pyrite (mg)** | 1; 2; 3; 4 ±0.02 | 1; 2 ±0.02 | 1; 2 ±0.02 |
| **Teneur en soufre pyritique (wt. % )** | 53±2 | 53±2 | 53±2 |

## Revendications

**1.** Procédé pour quantifier le soufre pyritique dans un échantillon de roche sédimentaire, dans lequel on applique au moins les étapes suivantes :

A. on chauffe ledit échantillon en atmosphère inerte, entre une première température comprise entre 80°C et 320°C et une deuxième température comprise entre 600°C et 700°C, en suivant une première séquence de températures, et on mesure en continu une quantité de composés hydrocarbonés, une quantité de CO et une quantité de $CO_2$ libérées pendant ladite première séquence de températures;

B. on oxyde en continu au moins une partie des effluents issus de ladite chauffe en atmosphère inerte dudit échantillon, on mesure en continu une quantité de $SO_2$ libérée par ladite oxydation desdits effluents en fonction du temps de ladite chauffe en atmosphère inerte, et on détermine au moins une teneur en soufre pyritique de pyrolyse $S_{Pyrol}^{Pyrit}$ à partir de ladite quantité de $SO_2$ ;

C. on chauffe en atmosphère oxydante le résidu dudit échantillon issu de ladite chauffe en atmosphère inerte entre une troisième température comprise entre 280°C et 320°C et une quatrième température supérieure ou égale à 800°C, en suivant une seconde séquence de températures, et on mesure en continu une quantité de CO et une quantité de $CO_2$ libérées pendant ladite seconde séquence de températures ;

**caractérisé en ce qu'**on détermine au moins une teneur en soufre pyritique $S^{Pyrit}$ contenu dans ledit échantillon à partir d'une formule du type :

$$S^{Pyrit} = S_{pyrol}^{Pyrit} * \frac{(1 + \dfrac{\beta}{1-\beta} + \dfrac{\gamma}{1-\gamma})}{\alpha}$$

où $\alpha$ est un paramètre représentant une proportion dudit soufre pyritique de pyrolyse par rapport audit soufre total, $\beta$ est un paramètre représentant un effet de la matrice minérale sur ladite proportion, $\gamma$ est un paramètre représentant un effet de la matrice organique sur ladite proportion, les valeurs desdits paramètres $\alpha$ et β étant prédéterminées, et ledit paramètre γ étant déterminé à partir d'une formule du type :

$$\gamma = f(\boldsymbol{OI}, \boldsymbol{HI})$$

où f est une fonction d'au moins un indice d'oxygène OI et d'un indice d'hydrogène HI, ledit indice d'hydrogène HI étant fonction au moins de ladite quantité de composés hydrocarbonés mesurée pendant ladite chauffe en atmosphère inerte et desdites quantités de CO et de $CO_2$ mesurées pendant lesdites première et seconde séquence de températures, et ledit indice d'oxygène OI étant fonction au moins desdites quantités de CO et de $CO_2$ mesurées pendant lesdites première et seconde séquences de températures, et dans lequel ladite fonction f est une combinaison linéaire dudit indice d'oxygène OI et dudit indice d'hydrogène HI s'exprimant selon une formule du type : $\gamma = a * OI + b * HI + c$ , où a, b et c sont des constantes prédéterminées.

**2.** Procédé selon la revendication 1, dans lequel ladite constante a est comprise entre 0.28 et 0.46, et vaut préférentiellement 0.37.

**3.** Procédé selon l'une des revendications 1 à 2, dans lequel ladite constante b est comprise entre -0.007 et -0.005 et vaut préférentiellement -0.006.

24

**4.** Procédé selon l'une des revendications 1 à 3, dans lequel ladite constante c est comprise entre 4.99 et 6.49 et vaut préférentiellement 5.74.

**5.** Procédé selon l'une des revendications précédentes, dans lequel on détermine ledit indice d'hydrogène HI selon une formule du type :

$$HI = \frac{100*S2}{TOC},$$

où

- S2 est une quantité de composés hydrocarbonés qui sont craqués au cours de la ladite première séquence de températures, S2 étant déterminée à partir de ladite quantité de composés hydrocarbonés libérés pendant ladite chauffe en atmosphère inerte,
- TOC est une teneur en carbone organique total dudit échantillon s'écrivant sous la forme *TOC(wt%) = PC + RC,* où PC est une teneur en carbone organique de pyrolyse dudit échantillon déterminée à partir desdites mesures de CO et $CO_2$ libérés pendant ladite première séquence de températures, et où RC est une teneur en carbone organique résiduelle dudit échantillon déterminée à partir desdites mesures de CO et de CO2 libérés pendant ladite seconde séquence de températures.

**6.** Procédé selon l'une des revendications précédentes, dans lequel dans lequel on détermine ledit indice d'oxygène OI selon une formule du type :

$$OI = \left[\frac{100*S3CO_2}{TOC}\right],$$

où :

- $S3CO_2$ est une quantité de $CO_2$ mesurée entre ladite première température de ladite première séquence de températures et une première température intermédiaire de ladite première séquence de températures comprise entre 350°C et 450°C, et valant préférentiellement 400°C ;
- TOC est une teneur en carbone organique total dudit échantillon et s'écrit *TOC(wt%) = PC + RC,* où PC est une teneur en carbone organique de pyrolyse dudit échantillon déterminée à partir desdites mesures de CO et CO2 libérés pendant ladite première séquence de températures, et où RC est une teneur en carbone organique résiduelle dudit échantillon déterminée à partir desdites mesures de CO et de CO2 libérés pendant ladite seconde séquence de températures.

**7.** Procédé selon l'une des revendications 5 ou 6, dans lequel ladite teneur en carbone organique de pyrolyse PC dudit échantillon est déterminée selon une formule du type :

$$PC(wt\%) = [Q * 0.083] + \left[(S3CO + \tfrac{1}{2}S3'CO) * \frac{12}{280}\right] + \left[S3CO_2 * \frac{12}{440}\right],$$

avec

- *S3CO2* est une quantité de $CO_2$ mesurée entre ladite première température de ladite première séquence de températures et une première température intermédiaire de ladite première séquence de températures comprise entre 350°C et 450°C, et valant préférentiellement 400°C ;
- *S3CO* est une quantité de CO mesurée entre ladite première température de ladite première séquence de températures et une deuxième température intermédiaire de ladite première séquence de températures comprise entre 500 et 600°C, et valant préférentiellement 550°C ;
- *S3'CO* est une quantité de CO mesurée entre ladite seconde température intermédiaire de ladite première séquence de températures et ladite deuxième température de ladite première séquence de températures ;

**8.** Procédé selon l'une des revendications 5 à 7, dans lequel ladite teneur en carbone organique résiduelle RC dudit échantillon est déterminée selon une formule du type :

$$RC(wt\%) = \left[S4CO_2 * \frac{12}{440}\right] + \left[S4CO * \frac{12}{280}\right],$$

où S4CO et S4CO$_2$ correspondent respectivement à une quantité de CO et de CO$_2$ mesurées entre ladite troisième température de ladite seconde séquence de températures et une température intermédiaire de ladite seconde séquence de températures comprise entre 600°C et 700°C, et valant préférentiellement 650°C.

**9.** Procédé selon l'une des revendications précédentes, dans lequel ledit échantillon est de type roche réservoir, et dans lequel ladite première température est comprise entre 100°C et 200°C.

**10.** Procédé selon l'une des revendications précédentes, dans lequel ledit échantillon est de type roche mère conventionnelle ou roche mère non conventionnelle immature, et dans lequel ladite première température est comprise entre 280°C et 320°C.

**11.** Procédé selon l'une des revendications précédentes, dans lequel ledit échantillon est de type roche mère non conventionnelle à gaz ou à huile, et dans lequel ladite première température est comprise entre 80°C et 120°C.

**12.** Procédé selon l'une des revendications précédentes, dans lequel ledit paramètre $\alpha$ est compris entre 0.40 et 0.46, et vaut préférentiellement 0.43.

**13.** Procédé l'une des revendications précédentes, dans lequel ledit échantillon de roche est de type argile, et pour lequel ledit paramètre β est compris entre 0.04 et 0.7, et vaut préférentiellement 0.38.

**14.** Procédé selon l'une des revendications 1 à 12, dans lequel ledit échantillon de roche est de type marne, et pour lequel ledit paramètre β est compris entre 0.7 et 0.9, et vaut préférentiellement 0.78.

**15.** Procédé selon l'une des revendications 1 à 12, dans lequel ledit échantillon de roche est de type calcaire, et pour lequel le paramètre β est compris entre 0.85 et 0.97, et vaut préférentiellement 0.9.

**16.** Procédé selon l'une des revendications précédentes, dans lequel on mesure en outre une quantité de SO$_2$ libérée pendant ladite seconde séquence de températures, on détermine au moins une teneur en soufre de pyrolyse $S_{Pyrol}$ à partir de ladite quantité de SO$_2$ mesurée pendant ladite première séquence de températures et une teneur en soufre d'oxydation $S_{Oxy}$ à partir de ladite quantité de SO$_2$ mesurée pendant ladite seconde séquence de températures, et on détermine une teneur en soufre organique $S^{Org}$ à partir au moins de ladite teneur en soufre pyritique $s^{Pyrit}$, de ladite teneur en soufre de pyrolyse $S_{Pyrol}$ et de ladite teneur en soufre d'oxydation $S_{Oxy}$.

**17.** Procédé selon la revendication 16, dans lequel ladite quatrième température est comprise entre 800°C et 900°C, et dans lequel, on détermine une teneur en soufre organique $S^{Org}$ selon la formule : $S^{Org} = S_{Pyrol} + S_{Oxy} - S^{Pyrit}$.

**18.** Procédé selon la revendication 16, dans lequel ladite quatrième température est supérieure à 1150°C, et est préférentiellement inférieure 1250°C, et dans lequel, on détermine en outre une teneur en soufres sulfates $S_{Oxy}^{Sulfa}$ à partir de la dite quantité de SO$_2$ mesurée pendant ladite seconde séquence de températures, et on en déduit une teneur en soufre organique selon la formule :

$$S^{Org} = S_{Pyrol} + S_{Oxy} - S^{Pyrit} - S_{Oxy}^{Sulfa}.$$

## Patentansprüche

**1.** Verfahren zur Quantifizierung von Pyritschwefel in einer Sedimentgesteinsprobe, wobei zumindest die folgenden Schritte angewandt werden:

A. die Probe wird in einer inerten Atmosphäre zwischen einer ersten Temperatur zwischen 80 °C und 320 °C und einer zweiten Temperatur zwischen 600 °C und 700 °C erhitzt, wobei eine erste Temperatursequenz befolgt wird, und eine Menge an Kohlenwasserstoffverbindungen, eine CO-Menge und eine CO$_2$-Menge, die während der ersten Temperatursequenz freigesetzt werden, werden kontinuierlich gemessen;

B. mindestens ein Teil der beim Erhitzen der Probe in einer inerten Atmosphäre gebildeten Abgase wird kontinuierlich oxidiert, eine $SO_2$-Menge, die durch die Oxidation der Abgase in Abhängigkeit von der Zeit des Erhitzens in einer inerten Atmosphäre freigesetzt wird, wird kontinuierlich gemessen, und mindestens ein Pyrolysepyritschwefelgehalt $S_{Pyrol}^{Pyrit}$ wird ausgehend von der $SO_2$-Menge bestimmt;

C. der Rückstand der Probe aus dem Erhitzen in einer inerten Atmosphäre wird in einer oxidierenden Atmosphäre zwischen einer dritten Temperatur zwischen 280 °C und 320 °C und einer vierten Temperatur von mehr als oder gleich 800 °C erhitzt, wobei eine zweite Temperatursequenz befolgt wird, und eine CO-Menge und eine $CO_2$-Menge, die während der zweiten Temperatursequenz freigesetzt werden, werden kontinuierlich gemessen; **dadurch gekennzeichnet, dass** mindestens ein in der Probe enthaltener Pyritschwefelgehalt $S^{Pyrit}$ ausgehend von einer Formel des Typs:

$$S^{Pyrit} = S_{pyrol}^{Pyrit} * \frac{(1 + \frac{\beta}{1-\beta} + \frac{\gamma}{1-\gamma})}{\alpha}$$

bestimmt wird, wobei $\alpha$ ein Parameter ist, der einen Anteil des Pyrolysepyritschwefels in Bezug auf den Gesamtschwefel darstellt, $\beta$ ein Parameter ist, der eine Wirkung der mineralischen Matrix auf den Anteil darstellt, $\gamma$ ein Parameter ist, der eine Wirkung der organischen Matrix auf den Anteil darstellt, wobei die Werte der Parameter $\alpha$ und $\beta$ vorbestimmt sind und der Parameter $\gamma$ ausgehend von einer Formel des Typs:

$$\gamma = f(OI, HI)$$

bestimmt wird, wobei $f$ eine Funktion mindestens eines Sauerstoffindex OI und eines Wasserstoffindex HI ist, der Wasserstoffindex HI eine Funktion zumindest der Menge an Kohlenwasserstoffverbindungen ist, die während des Erhitzens in einer inerten Atmosphäre gemessen werden, und die CO- und $CO_2$-Mengen, die während der ersten und der zweiten Temperatursequenz gemessen werden, und der Sauerstoffindex OI zumindest eine Funktion der CO- und $CO_2$-Mengen sind, die während der ersten und der zweiten Temperatursequenz gemessen werden, und wobei die Funktion $f$ eine lineare Kombination des Sauerstoffindex OI und des Wasserstoffindex HI ist, die durch eine Formel des Typs: $\gamma = a * OI + b * HI + c$ ausgedrückt wird, wobei a, b und c vorbestimmte Konstanten sind.

2. Verfahren nach Anspruch 1, wobei die Konstante a zwischen 0,28 und 0,46 liegt und vorzugsweise 0,37 beträgt.

3. Verfahren nach einem der Ansprüche 1 bis 2, wobei die Konstante b zwischen -0,007 und -0,005 liegt und vorzugsweise -0,006 beträgt.

4. Verfahren nach einem der Ansprüche 1 bis 3, wobei die Konstante c zwischen 4,99 und 6,49 liegt und vorzugsweise 5,74 beträgt.

5. Verfahren nach einem der vorhergehenden Ansprüche, wobei der Wasserstoffindex HI gemäß einer Formel des Typs:

$$HI = \frac{100*S2}{TOC},$$

bestimmt wird, wobei

- S2 eine Menge an Kohlenwasserstoffverbindungen ist, die im Verlauf der ersten Temperatursequenz gecrackt werden, wobei S2 ausgehend von der Menge an Kohlenwasserstoffverbindungen bestimmt wird, die beim Erhitzen in einer inerten Atmosphäre freigesetzt werden,
- TOC ein Gehalt an organischem Gesamtkohlenstoff der Probe ist, der in der Form *TOC (Gew. -%) = PC + RC* abgefasst ist, wobei PC ein Gehalt an organischem Kohlenstoff aus der Pyrolyse der Probe ist, der ausgehend von den Messungen von CO und $CO_2$ bestimmt wird, die während der ersten Temperatursequenz freigesetzt werden, und wobei RC ein Restgehalt an organischem Kohlenstoff der Probe ist, der ausgehend von den Messungen von CO und $CO_2$ bestimmt wird, die während der zweiten Temperatursequenz freigesetzt werden.

6. Verfahren nach einem der vorhergehenden Ansprüche, wobei der Sauerstoffindex OI gemäß einer Formel des Typs:

$$OI = \left[\frac{100*S3CO_2}{TOC}\right],$$

bestimmt wird, wobei:

- $S3CO_2$ eine $CO_2$-Menge ist, die zwischen der ersten Temperatur der ersten Temperatursequenz und einer ersten Zwischentemperatur der ersten Temperatursequenz zwischen 350 °C und 450 °C und vorzugsweise bei 400 °C gemessen wird;
- TOC ein Gehalt an organischem Gesamtkohlenstoff der Probe ist, der als *TOC(Gew.-%) = PC* + RC abgefasst ist, wobei PC ein Gehalt an organischem Kohlenstoff aus der Pyrolyse der Probe ist, der ausgehend von den Messungen von CO und $CO_2$ bestimmt wird, die während der ersten Temperatursequenz freigesetzt werden, und wobei RC ein Restgehalt an organischem Kohlenstoff der Probe ist, der ausgehend von den Messungen von CO und $CO_2$ bestimmt wird, die während der zweiten Temperatursequenz freigesetzt werden.

7. Verfahren nach einem der Ansprüche 5 oder 6, wobei der Gehalt PC an organischem Kohlenstoff aus der Pyrolyse der Probe gemäß einer Formel des Typs:

$$PC(wt\%) = [Q * 0.083] + \left[(S3CO + \frac{1}{2}S3'CO) * \frac{12}{280}\right] + \left[S3CO_2 * \frac{12}{440}\right]$$

bestimmt wird, wobei

- *S3CO2* eine $CO_2$-Menge ist, die zwischen der ersten Temperatur der ersten Temperatursequenz und einer ersten Zwischentemperatur der ersten Temperatursequenz zwischen 350 °C und 450 °C und vorzugsweise 400 °C gemessen wird;
- *S3CO* eine CO-Menge ist, die zwischen der ersten Temperatur der ersten Temperatursequenz und einer zweiten Zwischentemperatur der ersten Temperatursequenz zwischen 500 °C und 600 °C und vorzugsweise 550 °C gemessen wird;
- *S3'CO* eine CO-Menge ist, die zwischen der zweiten Zwischentemperatur der ersten Temperatursequenz und der zweiten Temperatur der ersten Temperatursequenz gemessen wird;

8. Verfahren nach einem der Ansprüche 5 bis 7, wobei der Restgehalt RC an organischem Kohlenstoff der Probe gemäß einer Formel des Typs:

$$RC(wt\%) = \left[S4CO_2 * \frac{12}{440}\right] + \left[S4CO * \frac{12}{280}\right],$$

bestimmt wird, wobei S4CO bzw. $S4CO_2$ einer CO- bzw. $CO_2$-Menge entsprechen, die zwischen der dritten Temperatur der zweiten Temperatursequenz und einer Zwischentemperatur der zweiten Temperatursequenz zwischen 600 °C und 700 °C und vorzugsweise bei 650 °C gemessen werden.

9. Verfahren nach einem der vorhergehenden Ansprüche, wobei die Probe vom Typ eines Speichergesteins ist und wobei die erste Temperatur zwischen 100 °C und 200 °C liegt.

10. Verfahren nach einem der vorhergehenden Ansprüche, wobei die Probe vom Typ eines konventionellen Muttergesteins oder eines unreifen unkonventionellen Muttergesteins ist und wobei die erste Temperatur zwischen 280 °C und 320 °C liegt.

11. Verfahren nach einem der vorhergehenden Ansprüche, wobei die Probe vom Typ eines unkonventionellen Gas- oder Öl-Muttergesteins ist und wobei die erste Temperatur zwischen 80 °C und 120 °C liegt.

**12.** Verfahren nach einem der vorhergehenden Ansprüche, wobei der Parameter $\alpha$ zwischen 0,40 und 0,46 liegt und vorzugsweise 0,43 beträgt.

**13.** Verfahren nach einem der vorhergehenden Ansprüche, wobei es sich bei der Gesteinsprobe um einen Tontyp handelt und wobei der Parameter $\beta$ zwischen 0,04 und 0,7 liegt und vorzugsweise 0,38 beträgt.

**14.** Verfahren nach einem der Ansprüche 1 bis 12, wobei es sich bei der Gesteinsprobe um einen Mergeltyp handelt und wobei der Parameter $\beta$ zwischen 0,7 und 0,9 liegt und vorzugsweise 0,78 beträgt.

**15.** Verfahren nach einem der Ansprüche 1 bis 12, wobei es sich bei der Gesteinsprobe um einen Kalktyp handelt und wobei der Parameter $\beta$ zwischen 0,85 und 0,97 liegt und vorzugsweise 0,9 beträgt.

**16.** Verfahren nach einem der vorhergehenden Ansprüche, wobei außerdem eine $SO_2$-Menge gemessen wird, die während der zweiten Temperatursequenz freigesetzt wird, mindestens ein Pyroloseschwefelgehalt $S_{Pyrol}$ ausgehend von der $SO_2$-Menge, die während der ersten Temperatursequenz gemessen wird, und ein Oxidationsschwefelgehalt $Soxy$ ausgehend von der $SO_2$-Menge, die während der zweiten Temperatursequenz gemessen wird, bestimmt werden, und ein Gehalt $S^{Org}$ an organischem Schwefel ausgehend zumindest vom Pyritschwefelgehalt $s^{Pyrit}$, dem Pyroloseschwefelgehalt $S_{Pyrol}$ und dem Oxidationsschwefelgehalt $S_{Oxy}$ gemessen wird.

**17.** Verfahren nach Anspruch 16, wobei die vierte Temperatur zwischen 800 °C und 900 °C liegt und wobei ein Gehalt $S^{Org}$ an organischem Schwefel gemäß der Formel: $S^{Org} = S_{Pyrol} + S_{Oxy} - S^{Pyrit}$ bestimmt wird.

**18.** Verfahren nach Anspruch 16, wobei die vierte Temperatur mehr als 1150 °C beträgt und vorzugsweise niedriger als 1250 °C ist und wobei außerdem ein Sulfatschwefelgehalt $S_{Oxy}^{Sulfa}$ ausgehend von der $SO_2$-Menge bestimmt wird, die während der zweiten Temperatursequenz gemessen wird, und indem ein Gehalt an organischem Schwefel gemäß der Formel: $S^{Org} = S_{Pyrol} + S_{Oxy} - S^{Pyrit} - S_{Oxy}^{Sulfa}$ abgezogen wird.

**Claims**

**1.** Process for quantifying the pyritic sulfur in a sedimentary rock sample, in which at least the following steps are applied:

A. said sample is heated in an inert atmosphere, between a first temperature of between 80°C and 320°C and a second temperature of between 600°C and 700°C, while following a first temperature sequence, and an amount of hydrocarbon-based compounds, an amount of CO and an amount of $CO_2$ released during said first temperature sequence are continuously measured;

B. at least one portion of the effluents resulting from said heating of said sample in an inert atmosphere is continuously oxidized, an amount of $SO_2$ released by said oxidation of said effluents as a function of the time of said heating in an inert atmosphere is continuously measured, and at least one pyrolysis pyritic sulfur content $S_{Pyrol}^{Pyrit}$ is determined from said amount of $SO_2$;

C. the residue of said sample resulting from said heating in an inert atmosphere is heated in an oxidizing atmosphere between a third temperature of between 280°C and 320°C and a fourth temperature of greater than or equal to 800°C, while following a second temperature sequence, and an amount of CO and an amount of $CO_2$ released during said second temperature sequence are continuously measured;

**characterized in that** at least one pyritic sulfur content $S^{Pyrit}$ contained in said sample is determined on the basis of a formula of the type:

$$S^{Pyrit} = S_{pyrol}^{Pyrit} * \frac{(1 + \frac{\beta}{1 - \beta} + \frac{\gamma}{1 - \gamma})}{\alpha}$$

wherein $\alpha$ is a parameter representing a proportion of said pyrolysis pyritic sulfur relative to said total sulfur, $\beta$

is a parameter representing an effect of the mineral matrix on said proportion, and $\gamma$ is a parameter representing an effect of the organic matrix on said proportion, the values of said parameters $\alpha$ and $\beta$ being predetermined, and said parameter $\gamma$ being determined from a formula of the type:

$$\gamma = f(\boldsymbol{OI}, \boldsymbol{HI})$$

wherein $f$ is a function of at least one oxygen index OI and of a hydrogen index HI, said hydrogen index HI being a function at least of said amount of hydrocarbon-based compounds measured during said heating in an inert atmosphere and said amounts of CO and of $CO_2$ measured during said first and second temperature sequences, and said oxygen index OI being a function at least of said amounts of CO and of $CO_2$ measured during said first and second temperature sequences, and in which said function $f$ is a linear combination of said oxygen index OI and of said hydrogen index HI which is expressed according to a formula of the type: $\gamma$ = $\boldsymbol{a}$ ∗ $\boldsymbol{OI}$ + $\boldsymbol{b}$ ∗ $\boldsymbol{HI}$ + $\boldsymbol{c}$, wherein a, b and c are predetermined constants.

2. Process according to Claim 1, in which said constant a is between 0.28 and 0.46, and is preferentially equal to 0.37.

3. Process according to either of Claims 1 and 2, in which said constant b is between -0.007 and -0.005, and is preferentially equal to -0.006.

4. Process according to one of Claims 1 to 3, in which said constant c is between 4.99 and 6.49, and is preferentially equal to 5.74.

5. Process according to one of the preceding claims, in which said hydrogen index HI is determined according to a formula of the type:

$$HI = \frac{100 * S2}{TOC},$$

wherein

- S2 is an amount of hydrocarbon-based compounds which are cracked during said first temperature sequence, S2 being determined from said amount of hydrocarbon-based compounds released during said heating in an inert atmosphere,
- TOC is a total organic carbon content of said sample which is written in the form $TOC(wt\%) = PC + RC$, wherein PC is an organic carbon content from pyrolysis of said sample determined from said measurements of CO and $CO_2$ released during said first temperature sequence, and wherein RC is a residual organic carbon content of said sample determined from said measurements of CO and of $CO_2$ released during said second temperature sequence.

6. Process according to one of the preceding claims, in which said oxygen index OI is determined according to a formula of the type:

$$OI = \left[\frac{100 * S3CO_2}{TOC}\right],$$

wherein:

- $S3CO_2$ is an amount of $CO_2$ measured between said first temperature of said first temperature sequence and a first intermediate temperature of said first temperature sequence of between 350°C and 450°C, and preferentially equal to 400°C;
- TOC is a total organic carbon content of said sample and is written $TOC(wt\%) = PC + RC$, wherein PC is an organic carbon content from pyrolysis of said sample determined from said measurements of CO and $CO_2$ released during said first temperature sequence, and wherein RC is a residual organic carbon content of said sample determined from said measurements of CO and of $CO_2$ released during said second temperature sequence.

7. Process according to either of Claims 5 and 6, in which said pyrolysis organic carbon content PC of said sample is determined according to a formula of the type:

$$PC(wt\%) = [Q * 0.083] + \left[(S3CO + \frac{1}{2}S3'CO) * \frac{12}{280}\right] + \left[S3CO_2 * \frac{12}{440}\right],$$

with

- S3CO2 is an amount of $CO_2$ measured between said first temperature of said first temperature sequence and a first intermediate temperature of said first temperature sequence of between 350°C and 450°C, and preferentially equal to 400°C;
- S3CO is an amount of CO measured between said first temperature of said first temperature sequence and a second intermediate temperature of said first temperature sequence of between 500 and 600°C, and preferentially equal to 550°C;
- S3'CO is an amount of CO measured between said second intermediate temperature of said first temperature sequence and said second temperature of said first temperature sequence.

8. Process according to one of Claims 5 to 7, in which said residual organic carbon content RC of said sample is determined according to a formula of the type:

$$RC(wt\%) = \left[S4CO_2 * \frac{12}{440}\right] + \left[S4CO * \frac{12}{280}\right],$$

wherein S4CO and S4CO_2 correspond respectively to an amount of CO and of $CO_2$ measured between said third temperature of said second temperature sequence and an intermediate temperature of said second temperature sequence of between 600°C and 700°C, and preferentially equal to 650°C.

9. Process according to one of the preceding claims, in which said sample is of reservoir rock type, and in which said first temperature is between 100°C and 200°C.

10. Process according to one of the preceding claims, in which said sample is of conventional source rock or immature shale play type, and in which said first temperature is between 280°C and 320°C.

11. Process according to one of the preceding claims, in which said sample is of gas-bearing or oil-bearing shale play type, and in which said first temperature is between 80°C and 120°C.

12. Process according to one of the preceding claims, in which said parameter $\alpha$ is between 0.40 and 0.46, and is preferentially equal to 0.43.

13. Process according to one of the preceding claims, in which said rock sample is of clay type, and for which said parameter $\beta$ is between 0.04 and 0.7, and is preferentially equal to 0.38.

14. Process according to one of Claims 1 to 12, in which said rock sample is of marl type, and for which said parameter $\beta$ is between 0.7 and 0.9, and is preferentially equal to 0.78.

15. Process according to one of Claims 1 to 12, in which said rock sample is of limestone type, and for which the parameter $\beta$ is between 0.85 and 0.97, and is preferentially equal to 0.9.

16. Process according to one of the preceding claims, in which, in addition, an amount of $SO_2$ released during said second temperature sequence is measured, at least one pyrolysis sulfur content $S_{Pyrol}$ is determined from said amount of $SO_2$ measured during said first temperature sequence and an oxidation sulfur content $S_{Oxy}$ is determined from said amount of $SO_2$ measured during said second temperature sequence, and an organic sulfur content $S^{Org}$ is determined from at least said pyritic sulfur content $S^{Pyrit}$, from said pyrolysis sulfur content $S_{Pyrol}$ and from said oxidation sulfur content $S_{Oxy}$.

17. Process according to Claim 16, in which said fourth temperature is between 800°C and 900°C, and in which an organic sulfur content $S^{Org}$ is determined according to the formula: $S^{Org} = S_{Pyrol} + S_{Oxy} - S^{Pyrit}$.

18. Process according to Claim 16, in which said fourth temperature is greater than 1150°C, and is preferentially less than 1250°C, and in which a sulfate sulfur content $S_{Oxy}^{Sulfa}$ is also determined from said amount of $SO_2$ measured during said second temperature sequence, and an organic sulfur content is deduced therefrom according to the formula: $$S^{Org} = S_{Pyrol} + S_{Oxy} - S^{Pyrit} - S_{Oxy}^{Sulfa}.$$

Figure 1a

Figure 1b

Figure 2

**Figure 3a**

**Figure 3b**

Figure 3c

**Figure 4**

**Figure 5a**

Figure 5b

Figure 5c

**Figure 5d**

**Figure 5e**

## RÉFÉRENCES CITÉES DANS LA DESCRIPTION

**Documents brevets cités dans la description**

- EP 2342557 A
- US 8796035 B
- FR 1759447
- EP 0691540 B1
- FR 3021748
- US 20150346179 A

**Littérature non-brevet citée dans la description**

- **ACHOLLA, F.V. ; ORR, W.L.** Pyrite removal from kerogen without altering organic matter: The chromous chloride method. *Energy Fuels,* 1993, vol. 7, 406-410
- **BEHAR, F. ; BEAUMONT, V. ; DE B. PENTEADO, H. L.** Rock-Eval 6 Technology: Performances and Developments. Oil & Gas Science and Technology. *Rev. IFP,* 2001, vol. 56, 111-134
- **BOLIN, T.B.** Direct determination of pyrite content in Argonne premium coals by the use of sulfur X-ray near edge absorption spectroscopy (S-XANES). *Energy and Fuels,* 2010, vol. 24, 5479-5482
- **CANFIELD, D.E. ; RAISWELL, R. ; WESTRICH, J.T. ; REAVES, C.M. ; BERNER, R.A.** The use of chromium reduction in the analysis of reduced inorganic sulfur in sediments and shales. *Chemical Geology,* 1986, vol. 54, 149-155
- **LANDAIS, P. ; MICHELS, R. ; BENKHEDDA, Z. ; KISTER, J. ; DEREPPE, J.-M.** Behavior of Oxidized Type II Kerogen during Artificial Maturation. *Energy and Fuels,* 1991, vol. 5, 860-866
- **ORR W.** Kerogen/asphaltene/sulfur relationships in sulfur-rich Monterey oils. *Org. Geochem.,* 1986, vol. 10, 499-516
- **VAIRAVAMURTHY, M.A. ; MALETIC, D. ; WANG, S. ; MANOWITZ, B. ; EGLINTON, T. ; LYONS, T.** Characterization of sulfur-containing functional groups in sedimentary humic substances by X-ray absorption near-edge structure spectroscopy. *Energy and Fuels,* 1997, vol. 11, 546-553
- **VANDENBROUCKE, M. ; LARGEAU, C.** Kerogen origin, evolution and structure. *Organic Geochemistry,* 2007, vol. 38, 719-833